# EUROPÄISCHE PATENTSCHRIFT

(11) **EP 1 212 338 B1**
(45) Veröffentlichungstag und Bekanntmachung des Hinweises auf die Patenterteilung: **21.11.2012**
(21) Anmeldenummer: 00934905.1
(22) Anmeldetag: 07.04.2000
(51) Int. Cl.: C07H 19/06, C07H 19/16, C12Q 1/68

(54) **Nucleosid-Derivate mit photolabilen Schutzgruppen**
Nucleoside derivatives with photolabile protective groups
Dérivés de nucléosides avec des groupes protecteurs photolabiles

(30) Priorität: 08.04.1999 DE 19915867; 28.01.2000 DE 10003631
(43) Veröffentlichungstag der Anmeldung: 12.06.2002
(73) Patentinhaber: Deutsches Krebsforschungszentrum Stiftung des öffentlichen Rechts, 69120 Heidelberg (DE)
(72) Erfinder: BEIER, Markus, D-69120 Heidelberg (DE); HOHEISEL, Jörg, D-69168 Wiesloch (DE)
(74) Vertreter: Schüssler, Andrea
(86) Internationale Anmeldenummer: PCT/DE2000/001148
(87) Internationale Veröffentlichungsnummer: WO 2000/061594

(56) Entgegenhaltungen:
- WO-A-94/10128
- WO-A-99/05315
- US-A- 5 763 599
- WEI ET AL: "A photoactivated prodrug", BIOORGANIC & MEDICINAL CHEMISTRY LETTERS, vol. 8, 1999, pages 2419-2422, XP001040668,

## Beschreibung

Gegenstand der vorliegenden Erfindung sind Nucleosid-Derivate mit photolabilen Schutzgruppen, Verfahren zu deren Herstellung, deren Verwendung sowie daraus aufgebaute Nucleinsäure-Chips.

Photolabile Schutzgruppen für die Hydroxy- und Phosphatfunktionen in Nucleosiden bzw. Nucleotiden sind von Bedeutung, da sie sich für die lichtgesteuerte Parallel-Synthese von Oligonukleotiden auf einer soliden Trägeroberfläche eignen (Fodor et al., Science 1991, 251, S. 767 ff.). Hiermit können Oligonukleotide oder Nukleinsäure-Chips aufgebaut werden, die z.B. für eine effiziente Sequenzierung von Nukleinsäuren eingesetzt werden können.

In der WO-A-94/10128 werden ausschließlich Derivate mit Photoschutzgruppen vom Benzyl-Typ ausgewiesen.

In der US-A-5,763,599 werden nur Verbindungen beschrieben, bei denen sich die Photoschutzgruppen an der 5'-O-Position und nicht an der 3'-O-Position befinden. Alle in dieser Druckschrift aufgeführten Verbindungen sind Derivate der NPPOC-Gruppe.

Auch in "Wei et al., Bioorganic & Medicinal Chemistry Lett. 8 (1998), S. 2419-2422" wird in Scheme 1 nur eine Verbindung beschrieben, bei der sich die Photoschutzgruppe an der 5'-O-Position und nicht an der 3'-O-Position befindet. Dieser Artikel ist auf ein ganz spezielles Problem gerichtet, nämlich photolabile Derivate von 5'-Fluorodeoxyuridin zu designen und zu synthetisieren.

In der WO-A-99/05315 wird ein Verfahren zur festphasengestützten Sequenzierung einer Polynucleotidsequenz beschrieben. Hierbei wird die zu bestimmende Polynukleotidsequenz mit einer an die Festphase gekoppelten Polymerase unter Zugabe von Nucleotid-Reagenzien zur Reaktion gebracht. Bei den verwendeten Nucleotid-Reagenzien handelt es sich um Triphosphat-Reagentien, die an der 3'-O-Position eine Schutzgruppe aufweisen. Der Zugang zu der 3'-O-photolabil geschützten 5'-Triphosphat-Verbindung ausgehend von der 3'-ungeschützten 5'-Triphosphat-Verbindung wird beschrieben.

Bislang sind einzig photolithografische Herstellungsverfahren von DNA-Chips unter Verwendung von 3'-O-Phosphitamiden, die entsprechend die temporäre photolabile Schutzgruppe an der 5'-O-Position aufweisen, bekannt (WO-A-96/18634). Unter Verwendung dieser Nucleinsäurebausteine lassen sich DNA-Chips herstellen, wobei der Aufbau des Oligomers vom 3'- zum 5'-Ende erfolgt. Das fertiggestellte Oligomer ist somit über das 3'-O-Ende an der festen Phase verankert, das S'-OH-Ende ist frei zugänglich. DNA-Chips, die mit dieser Methode erzeugt wurden, lassen sich für Hybridisierungsexperimente verwenden, aber nicht für bestimmte Enzymreaktionen (z.B. mit der der DNA-Polymerase oder Ligase), die ein freies 3'-OH erfordern.

Die Aufgabe der vorliegenden Erfindung besteht deshalb darin, 3'-photolabile Nucleoside und deren Derivate bereitzustellen und mit den daraus gewonnenen 3'-photolabilen Nucleosiden Nukleinsäure-Chips zu generieren, bei denen die über die lichtgesteuerte Synthese aufgebauten Oligomeren über das 5'-Ende an die feste Phase gekoppelt sind und somit Enzymreaktionen am 3'-Ende möglich machen.

Diese Aufgabe wird erfindungsgemäß durch die Nucleosid-Derivate der allgemeinen Formel (I) entsprechend Patentanspruch 1 gelöst. Vorteilhafte Ausgestaltungen ergeben sich aus den Unteransprüchen.

Erfindungsgemäße Nucleosid-Derivate haben folgende Formel: 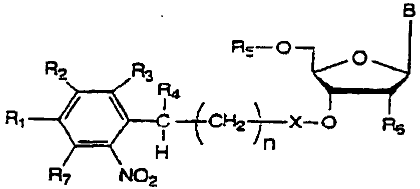 mit
R¹ = H, NO₂, CN, OCH₃, Halogen, Alkyl-, Alkoxy- oder Alkoxyalkylrest mit 1 bis 4 C-Atomen oder ein ggf. substituierter Arylrest oder ein aliphatischer Acylrest mit 2 bis 5 Atomen
R² = H, NO₂, CN, OCH₃, Halogen, Alkyl-, Alkoxy- oder Alkoxyalkylrest mit 1 bis 4 C-Atomen oder ein ggf. substutierter Arylrest oder ein aliphatischer Acylrest mit 2 bis 5 Atomen,
R³ = H, NO₂, CN, OCH₃, Halogen Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁴ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁵ = H, Dimethoxytrityl (DMTr) oder eine in der Nucleotidchemie übliche Schutzgruppe oder eine übliche Schutzgruppe zur Herstellung von Oligonukleotiden
R⁸ = H, OH, Halogen oder ΨR⁸, wobei Ψ = O oder S und R⁸ = Alkyl- oder Alkoxyalkyl mit 1 bis 4 C-Atomen oder ein ggf. substituierter Arylrest oder ein aliphatischer Acylrest mit 2 bis 5 Atomen sowie eine in der Nukleotidchemie übliche Schutzgruppe
R⁷ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
X = SO₂, OC(O) oder OC(S)
X = SO₂, OC(O) oder OC(S)
n = 0 oder 1 für X = SO₂
n = 1 für X = OC(O) oder OC(S)
B = H, Adenin, Cytosin, Guanin, Thymin, Uracil, 2,6-Diaminopurin-9-yl,
Hypoxanthin-9-yl, 5-Methylcytosin-1-yl, 5-Amino-4-Imidazolcarbonsäure-1-yl oder 5-Amino-4-Imidazolcarbonsäuroamid-3-yl, wobei im Falle von B = Adenin, Cytosin oder Guanin die primäre Aminofunktion ggf. eine temporäre oder permanente Schutzgruppe aufweist bzw. Thymin oder Uracil an der O4-Position ggf. eine permanente Schutzgruppe aufweist.

Ein weiterer Gegenstand der vorliegenden Erfindung ist die Verwendung eines Nucleosid-Derivats mit photolabilen Schutzgruppen der allgemeinen Formel (I) 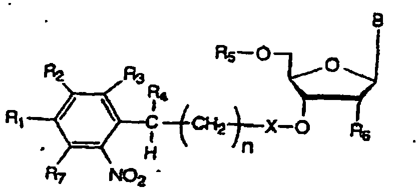 mit
R¹ = H, NO₂, CN, OCH₃, Halogen, Alkyl-, Alkoxy- oder Alkoxyalkylrest mit 1 bis 4 C-Atomen oder ein ggf. substituierter Arylrest oder ein aliphatischer Acylrest mit 2 bis 5 Atomen
R² = H, NO₂, CN, OCH₃, Halogen, Alkyl-, Alkoxy- oder Alkoxyalkylrest mit 1 bis 4 C-Atomen oder ein ggf. substutierter Arylrest oder ein aliphatischer Acylrest mit 2 bis 5 Atomen,
R³ = H, , NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁴ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁵ = H, Dimethoxytrityl oder eine in de Nucleotidchemie übliche Schutzgruppe oder eine übliche Schutzgruppe zur Herstellung von Oligonukleotiden
R⁸ = H, OH, Halogen oder ΨR⁸, wobei Ψ = O oder S und R⁸ = Alkyl- oder Alkoxyalkyl mit 1 bis 4 C-Atomen oder ein ggf. substituierter Arylrest oder ein aliphatischer Acylrest mit 2 bis 5 Atomen sowie eine in der Nukleotidchemie übliche Schutzgruppe
R⁷ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
n = 0 oder 1
X = SO₂, OC(O), OC(S)
B = H, Adenin, Cytosin, Guanin, Thymin, Uracil, 2,6-Diaminopurin-9-yl, Hypoxanthin-9-yl, 6-Methylcytosin-1-yl, 5-Amino-4-Imidazolcarbonsäure-1-yl oder 5-Amino-4-Imidazolcarbonsäureamid-3-yl, wobei im Falle von B = Adenin, Cytosin oder Guanin die primäre Aminofunktion ggf. eine temporäre oder permanente Schutzgruppe aufweist bzw. Thymin oder Uracil an der O4-Position ggf. eine permanente Schutzgruppe aufweist,
zum Aufbau von Oligonukleotiden oder Nukleinsäure-Chips.

Die Alkyl-, Alkoxy- oder Alkoxyalkylgruppe der Reste R¹, R², R³, R⁴ und R⁷ kann linear oder verzweigt sein, substituiert (insbesondere mit einem oder mehreren Halogenatomen) oder unsubstituiert sowie gesättigt oder ungesättigt sein. Zwischen den Resten kann eine Brückenverbindung bestehen, z.B. über eine Methylengruppe, so daß sich eine weitere Ringfunktion ergibt. Analoges gilt für die Acyl- oder Arylgruppe der Reste R², R³, R⁴ oder R⁷. Hier kommen als Substituenten neben Halogenatomen auch Alkylgruppen in Frage. Bevorzugte Alkylreste sind Methyl-Ethyl-, n-Propyl-, n-Butyl-, iso-Propyl, tert-Butyl-. Bevorzugte Alkoxyreste sind die Methoxy-, Ethoxy- oder tert-Butoxygruppierung. Bevorzugte aliphatische Acylreste sind der Formylrest (-CHO), Acetylrest (-CO-CH₃), Propionylrest (-CO-C₂M₃) oder Butyrylrest (-CO-C₃H₇). Bevorzugte (Hetero)Arylreste sind der Phenyl-, Thienyl-, Thiophenyl-, Furyl-, Furanyl-, Pyranyl-, Pyrrolyl-, Imidazolyl-, Pyrazolyl-, Pyridyl-, Pyrazinyl-, Pyrimidinyl-, Pyrazinyl-, Pyridazinyl-, Thiazolyl-, Oxazolyl-, Indolyl-, Pyrrolinyl-, Imidazolinyl-, Pyrazolinyl-, Thiazolinyl-, Triazolyl-, Tetrazolylgruppe sowie sich daraus ergebende annellierte Ringe.

Vorzugsweise stellt R⁴ H oder einen Methylrest dar. Im Falle von R⁴ ≠ H sind die Substituenten R¹ - R³ am Phenylring vorzugsweise Wasserstoffreste. Außerdem stellt im Falle von R² = OCH₃ R³ vorzugsweise einen Wasserstoffrest dar.

In der Position R⁵ bedeutet "eine bei der Herstellung von Oligonukleotiden übliche Schutzgruppe" beispielsweise eine Phosphitamid-Gruppe, wie NC-CH₂-CH₂-O-P-N(Q)₂, p-NC-C₆H₄-CH₂-CH₂-O-P-N(Q)₂, p-NO₂-C₆H₄-CH₂-CH₂-O-P-N(Q) ₂ oder CH₂=CH-CH₂-O-P-N(Q)₂, wobei die Q-Gruppen gleich oder verschieden sein können und lineare oder verzweigte Alkylreste mit 1 bis 4 C-Atomen, vorzugsweise Ethyl- oder Isopropylreste, bedeuten.

In der Position R⁶ bedeutet "eine in der Nukleotidchemie übliche Schutzgruppe" (=R⁸) insbesondere H sowie die üblichen O-Alkyl-, O-Alkenyl-, O-Acetal- oder O-Silylether-Schutzgruppen. Bevorzugte Schutzgruppen sind O-Methyl- oder O-Ethylreste, O-Allylreste, O-Tetrahydropyranyl- bzw. O-Methoxytetrahydropyranyl-Reste sowie O-t-Butyldimethylsilyl-Reste.

Die an den Basen B ggf. permanent vorkommenden Schutzgruppen basieren vorzugsweise auf Acyl-Schutzgruppen. Bevorzugt sind vor allem Phenoxyacetyl-, tert-Butylphenoxyacetyl-, Isobutyryl-, Acetyl-, Benzoyl-, Allyloxycarbonyl-, Phthaloyl-, Dansylethyloxycarbonyl-, 2-(4-Nitrophenyl)ethoxycarbonyl- oder Dimethylformamidino-Reste. Im Falle von Adenin, Cytosin und Guanin handelt es sich vorzugsweise um Phenoxyacetyl-, tert-Butylphenoxyacetyl, Acetyl- oder 2-(4-Nitrophenyl)ethhoxycarbonyl-Gruppen zum Schutz der exocyclischen Aminofunktionen. Die O⁶-Position von Guanin kann ggf. durch eine Schutzgruppe wie 2-(4-Nitrophenylsulfonyl)ethyl- oder 2-(4-Nitrophenyl)ethyl- geschützt sein. Ebenso kann die O⁴-Position von Thymin oder Uracil eine Schutzgruppe wie 2-(4-Nitrophenylsulfonyl)ethyl- oder 2-(4-Nitrophenyl)ethyl- aufweisen.

Halogen bedeutet erfindungsgemäß F, Cl, Br, I, wobei die drei letztgenannten bevorzugt sind.

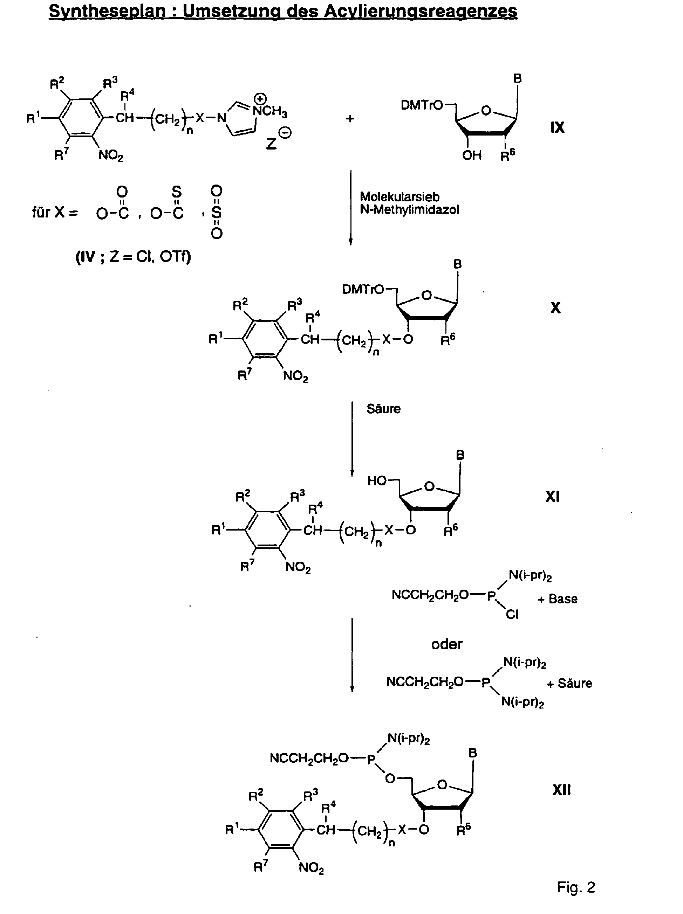

Die Herstellung der erfindungsgemäßen Nucleosid-Derivate ist beispielhaft in Fig. 2 gezeigt, worauf nachfolgend Bezug genommen wird. Die Erwähnung von bestimmten Halogen- und Alkylsubstitutionen schließt immer gleichwirkende Äquivalente ein, z.B. "Chlor-" schließt nicht aus, daß auch die entsprechenden Iod- oder Bromverbindungen einsetzbar sind. Ebensolches gilt für "Methyl-", das auch die entsprechenden anderen Niederalkylverbindungen, wie Ethyl-, Propyl- oder Butyl mit einschließt. Die Reste R¹, R², R³, R⁴, R⁶, R⁷ und X haben die oben genannten Bedeutungen.

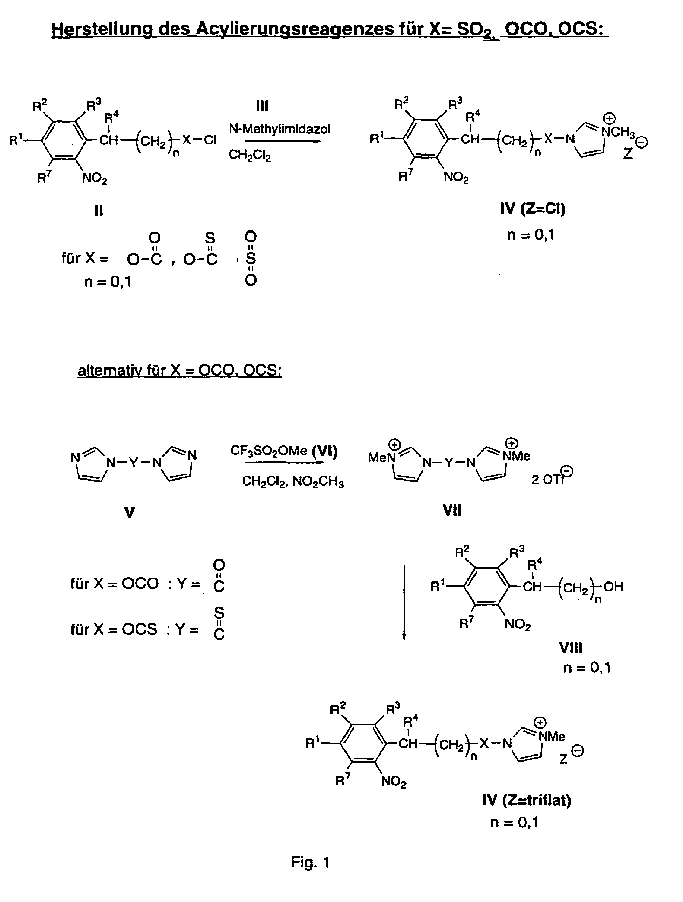

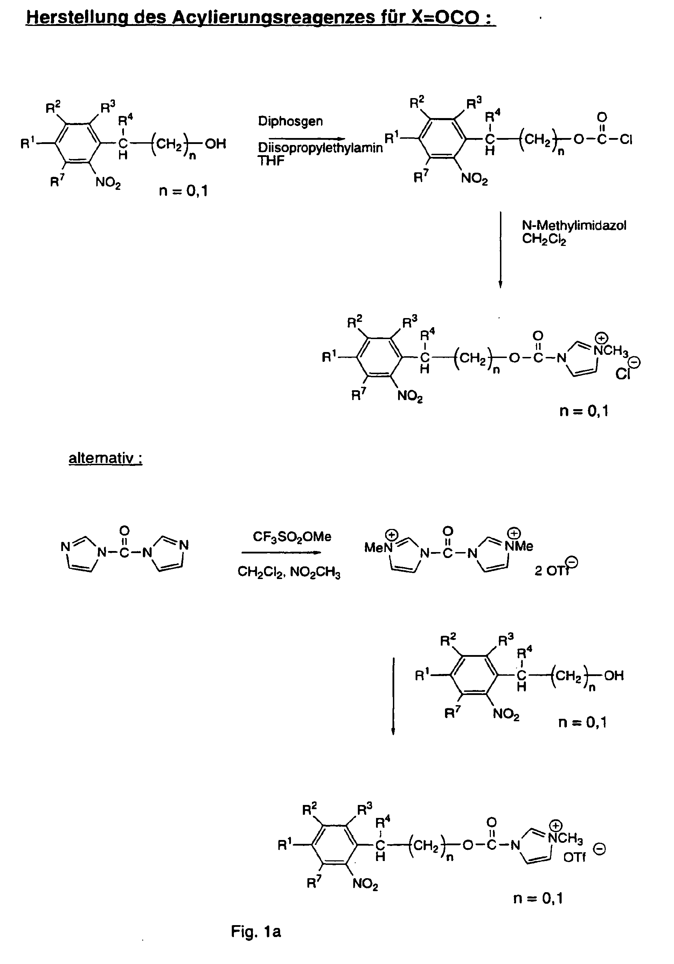

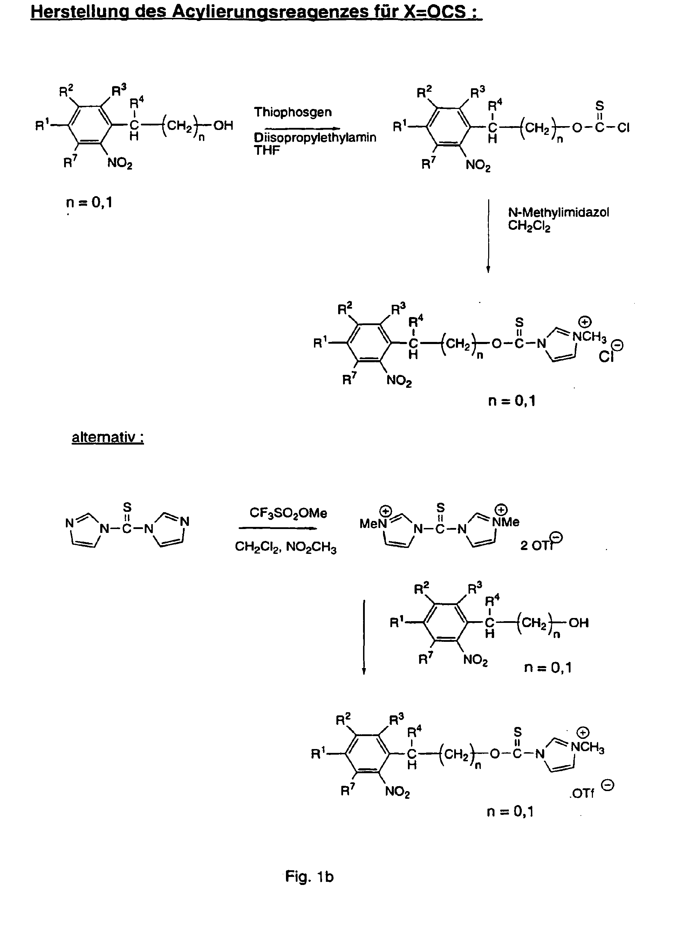

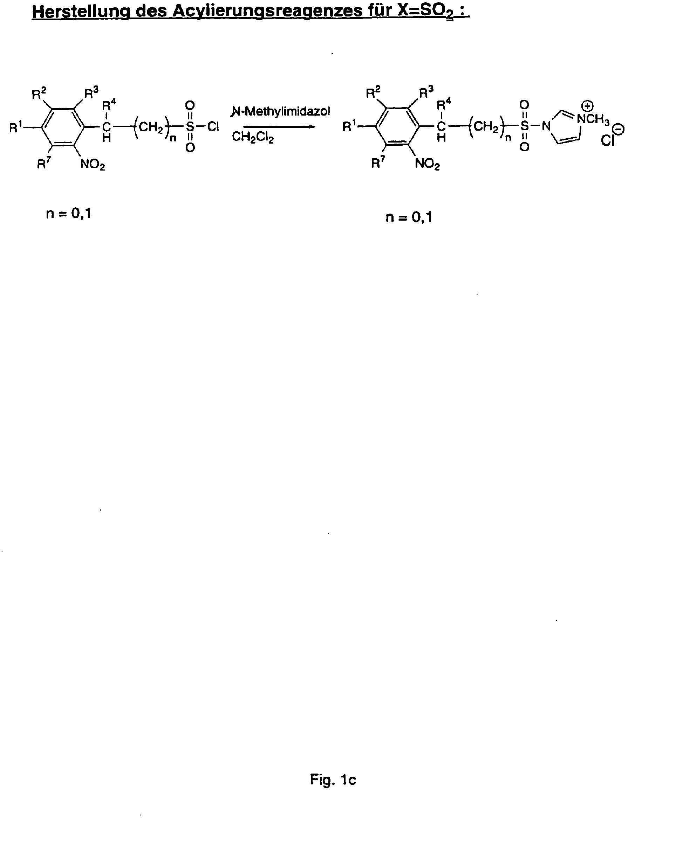

Die Herstellung beginnt mit der Präparation eines Acylierungsreagenzes. Hierzu wird auf Fig. 1 verwiesen. Ausgegangen wird hierfür bevorzugt von einem Chlorkohlensäureester (II, mit X = OCO), der sich beispielsweise gemäß der Vorschrift in WO-A-96/18634 oder gemäß nachfolgendem Beispiel 1 erhalten läßt. Analog ist ein gewünschter Chlorthiokohlensäureester (II, mit X = OCS) über die analoge Umsetzung mit Thiophosgen zugänglich. Das Acylierungsreagenz (IV) wird dann durch Umsetzung des Chlorkohlensäureesters (II, mit X = OCO) oder des Chlorthiokohlensäureesters (II, mit X = OCS) oder eine entsprechendes Sulfonylchlorid-Derivat (II, X = SO₂) mit einer Verbindung (III), vorzugsweise N-Methylimidazol, generiert. Diese Reaktionen werden in einem polaren organischen Lösungsmittel, vorzugsweise Dichlormethan, bei Temperaturen zwischen -10°C und + 10°C, vorzugsweise bei 0°C, durchgeführt. Vorzugsweise wird der Reaktion Molekularsieb zugesetzt und mit einem Überschuß an Verbindung (III), bevorzugt N-Methylimidazol, in bezug auf die eingesetzte Verbindung (II) gearbeitet: 1-10 Äquivalente, bevorzugt 2-5 Äquivalente. Alternativ zu N-Methylimidazol kann die Generation des Acylierungsreagenzes auch mittels anderer heterocyclischer Verbindungen (III), wie Pyridin, 4-N,N-Dimethylaminopyridin (DMAP), Triazol, Tetrazol oder Imidazol verlaufen.

Alternativ ist das Acylierungsreagenz (IV, Z = triflat) ausgehend von N,N-Carbonyldiimidazol (V, Y = CO) oder N,N-Thiocarbonyldiimidazol (V, Y = CS) nach Methylierung mit einem Methylierungsreagenz (VI), vorzugsweise Trifluormethansulfonsäuremethylester, und Umsetzung mit dem entsprechenden Alkohol (VIII) zugänglich. Hierbei wird die Reaktion bevorzugt in einem polaren organischen Lösungsmittel, vorzugsweise in Nitromethan oder einem Gemisch von Nitromethan und Dichlormethan, bei Temperaturen zwischen -10 und +10°C, vorzugsweise 0°C, durchgeführt. Die Methylierung von (V) erfolgt beispielsweise gemäß Rapoport et al., J. Am. Chem. Soc. 1989, 111, S. 4856-4859. Nach erfolgter Methylierung wird der entsprechende Alkohol (VIII) zugesetzt und somit das Acylierungsmittel vom Imidazoliumtyp (IV, Z = triflat) in Form eines Triflatsalzes generiert. Werden bei der Methylierung von (V) als Methylierungsreagenz (VI) Methyljodid oder Meerweinsalze eingesetzt, können die Acylierungsreagenzien (IV) entsprechend in Form ihrer Iodid oder Tetrafluoroborat-Salze erzeugt werden. Die Umsetzung von Verbindung (V) mit dem entsprechenden Methylierungsmittel erfolgt vorzugsweise im Verhältnis 1:1 bis 1:10, bevorzugt im Verhältnis 1:2. Die Umsetzung der methylierten Form (VI) mit dem entsprechenden Alkohol (VIII) erfolgt vorzugsweise im Verhältnis 1:1 bis 1:10, ganz bevorzugt im Verhältnis 1:1 bis 1:2.

Das Acylierungsreagenz (IV) wird weiter mit einem ggf. geschützten Nucleosid (IX) umgesetzt. 5'-DMTr-geschützte (DMTr = Dimethoxytrityl) Nucleoside der allgemeinen Formel (IX) sind beispielsweise käuflich erhältlich von den Firmen Proligo, Fluka, Sigma oder Aldrich.

Die Umsetzung des Acylierungsreagenz (IV) mit den geschützen Nukleosiden (IX) erfolgt vorzugsweise in Dichlormethan oder einem Lösungsmittelgemisch aus Dichlormethan und einem polaren organischen Lösungsmittel ggf. in Gegenwart einer Base, wie Pyridin, N-Methylimidazol, 4,N,N-Dimethylaminopyridin, Ethyldiisopropylamin (EtN(i-pr)₂) oder Triethylamin, bei Temperaturen zwischen -60 und +25°C, bevorzugt 0°C. Als polares organisches Lösungsmittel wird vorzugsweise Dichlorethan, Nitromethan, DMF oder Pyridin eingesetzt. Das Mischungsverhältnis von Dichlormethan zu dem polaren organischen Lösungsmittel unterliegt keiner Beschränkung. Vorzugsweise werden jedoch 1 bis 3 Vol.-Teile Dichlormethan pro Vol.-Teil polarem organischem Lösungsmittel eingesetzt. Bevorzugt wird eine Lösung des Acylierungsreagenz (IV) in Dichlormethan vorgelegt und das Nucleosid (IX), welches ebenso in Dichlormethan gelöst wurde, zugetropft. Das Molverhältnis von Acylierungsreagenz zu Nucleosid kann vorzugsweise zwischen 1:1 bis 5:1, bevorzugt bei 3:1, ganz bevorzugt bei 2:1 liegen, d.h. das Acylierungsreagenz wird bevorzugt im Überschuß verwendet. Die Konzentration des Nucleosids im Lösungsmittelgemisch unterliegt keiner Beschränkung. Sie liegt jedoch bevorzugt im Bereich von 0,1 bis 3,0 mmol pro 10 ml Lösungsmittel.

Nach erfolgter Umsetzung (bevorzugte Reaktionszeit: 1-12 Std.) kann das erhaltene Nucleosid-Derivat (X) isoliert werden. Danach erfolgt das Abspalten der 5'-Schutzgruppe am Nucleosidbestandteil durch Umsetzen mit bevorzugt Trichloressigsäure oder Toluolsulfonsäure, ggf. mit Camphersulfonsäure oder Dichloressigsäure in Dichlormethan. Es wird das Nucleosid-Derivat (XI) erhalten, das Formel (I) gehorcht.

Falls es gewünscht ist, kann an der 5'-Position des Nucleosid-Derivats (XI) eine Phosphitamid-Gruppe eingeführt werden. Dies geschieht beispielsweise durch die Umsetzung des Nucleosid-Derivats (XI) mit Bis (diisopropylamino) (ß-cyanoethoxy) phosphin unter Zusatz eines leicht aciden Katalysators (beispielsweise Tetrazol, Pyridin-Hydrochlorid) oder durch Umsetzung des Nucleosidderivats mit Chlor-Diisopropylamino-2-cyanoethoxy)phosphin unter Zusatz einer Base (z.B Diisopropylethylamin, N-Methylmorphin, Lutidin oder Collidin) und einem Lösungsmittel (z.B. THF, Dichlormethan). Dabei entsteht Verbindung (XII).

Der Vorteil die Umsetzung des geschützten Nucleosids mit einem milden Acylierungsreagenz durchzuführen, liegt in der Selektivität der Reaktion. Es werden quantitative Acylierungen der 3'-O-position des Nucleosidbausteins ohne nachteilige Nebenproduktformation erhalten. Werden hierzu reaktivere Acylierungsreagenzien, wie z.B. der entsprechende Chlorkohlensäureester selbst, verwendet, tritt eine unkontrollierte Reaktion ein. Es werden eine große Anzahl von Nebenprodukten gebildet, d.h. es besteht hierbei keinerlei Selektivität für das gewünschte 3-monoacylierte Produkt. In einer ganz besonders bevorzugten Ausführungsform wird das geschützte Nucleosid mit dem Acylierungsreagenz unter Zusatz von Molekularsieb durchgeführt. Mit Molekularsieb ist eine Steigerung der Selektivität für das gewünschte 3'-monoacylierte Produkt zu beobachten.

Die erfindungsgemäßen 3'-photolabilen Nucleoside können bei der photolithografischen Nukleinsäurechip-Synthese eingesetzt werden. Verfahren hierzu sind dem Fachmann ausreichend bekannt (z.B. Fodor et al., s.o). Ein geeignetes Verfahren hierzu ist beispielsweise auch in der deutschen Anmeldung DE 198 58 440.7 gezeigt. Dort wird zwar ein Verfahren gezeigt, das von 5'-photolabilen Nucleosiden ausgeht, aber die dort gezeigte Methodik läßt sich auf die Verwendung von 3'-photolabilen 5'-Phosphitamiden (gemäß Formel XII von Fig. 2) analog übertragen. Bei diesem Verfahren wird der bei der Chip-Synthese übliche Bestrahlungsschritt in Anwesenheit einer Base durchgeführt wird. Dieses Verfahren zur photolithografischen Biochip-Synthese bietet den Vorteil, daß eine effiziente Abspaltung von photolabilen Schutzgruppen stattfindet.

Unter einem Nucleinsäurechip sollen erfindungsgemäß auf einem Träger aufgebaute Biomoleküle, wie DNA oder RNA, sowie Nucleinsäureanaloga, wie PNA, LNA oder Chimären von diesen mit DNA, RNA oder untereinander verstanden werden.

Erfindungsgemäß ist jegliche(r) auf diesem Gebiet übliche Träger bzw. Matrix bei der Nucleinsäurechip-Herstellung einsetzbar. Dies sind insbesondere Glas, Folien bzw. Membranen aus Polypropylen, Nylon, Cellulose, Cellulosederivate (z.B. Celluloseacetat, Cellulose-Mischester), Polyethersulfonen, Polyamiden, Polyvinylchlorid, Polyvinylidenfluorid, Polyester, Teflon oder Polyethylen. Die Trägeroberflächen können auch mit freien oder geschützten funktionellen Gruppen versehen sein, z.B. eine Amino-Gruppe, Hydroxyl-Gruppe, Carboxyl-Gruppe, Carbonyl-Gruppe, Thiol-, Amid- oder Phosphat-Gruppe tragen. In einer bevorzugten Ausführungsform weisen die Trägeroberflächen eine Derivatisierung gemäß der deutschen Patentanmeldung 198 53 242.3 auf.

Bei dem oben genannten bevorzugten Verfahren zur photolithografischen Biochip-Synthese gemäß der deutschen Anmeldung DE 198 58 440.7 werden die Schritte Kondensation, Oxidation und Capping wie üblich (Fodor et al., Science 1991, 251, S. 767 ff.) durchgeführt. Allerdings findet der erste Schritt der Synthese, nämlich die Bestrahlung, unter Zusatz von Basen, bevorzugt starken Basen, insbesondere nicht-nukleophilen Basen, statt, was in Zusammenwirken mit dem bei der Bestrahlung angewendeten Licht zu einer überraschend effektiven Abspaltung der Schutzgruppen führt. Als Basen eignen sich die dem Fachmann bekannten Basen, wie z.B. DBU (1,8-Diazabicyclo[5.4.0]undec-7-en, DBN (1,5-Diazabicyclo[4.3.0]non-5-en, Diisoproylethylamin, Pyridin, Piperidin, Triethylamin, Diisopropylamin, N-Methylmorpholin, 2,6-Lutidin, Collidin, N-Methylimidazol, Dabco, N,N,-Dimethylaminopyridin. Die Bestrahlung kann unter den üblichen Bedingungen stattfinden. Die Wellenlänge der Bestrahlung ist von der verwendeten Schutzgruppe abhängig. Die geeigneten Wellenlängen sind dem Fachmann bekannt. Die Menge an während der Bestrahlung anwesender Base variiert zwischen 0,01 M und 1,0 M und ist natürlich von der Basenstärke abhängig. So hat sich es sich bewährt 0,03 bis 1 M (bevorzugt 0,05 bis 0,5 M) DBU in Acetonitril, 0,03 bis 0,8 M (bevorzugt 0,05 M) Diisoproylethylamin in Acetronitril oder 0,03 bis 1 M (bevorzugt 0,05 M) Piperidin in Acetonitril zu verwenden.

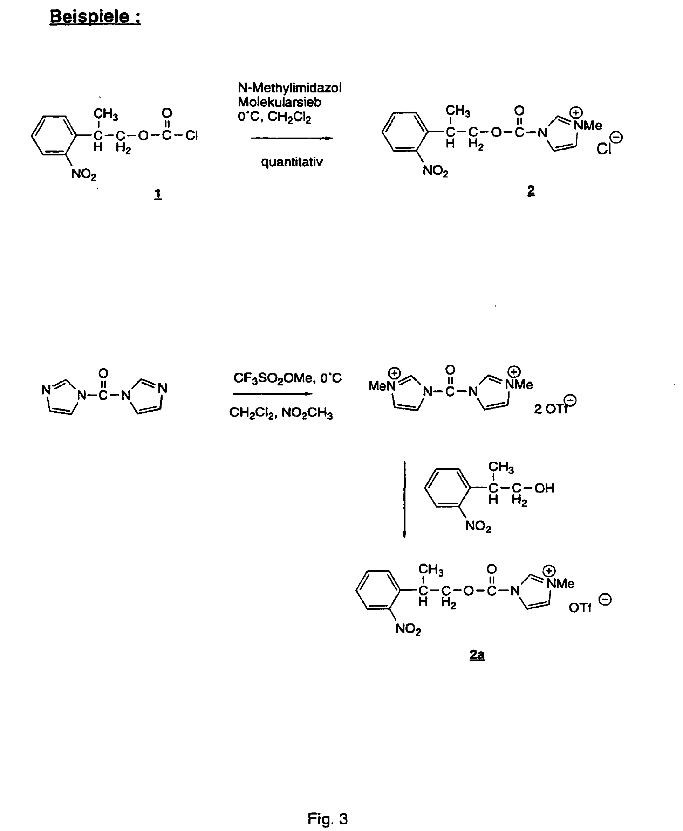

Nucleinsäure-Chips, die unter Verwendung erfindungsgemäßer Nucleoside hergestellt worden sind, sind dadurch gekennzeichnet, daß das fertiggestellte Oligomer mit der 5'-Position mit der festen Phase verbunden ist, das 3'-OH aber frei zugänglich ist (vgl. Fig. 3). DNA-Chips, die mit dieser Methode erzeugt wurden, lassen sich sowohl für Hybridisierungsexperimente als auch für bestimmte Enzymreaktionen (z.B. DNA-Polymerase), die ein freies 3'-OH erfordern, verwenden. Somit haben Nucleinsäure-Chips (bevorzugt DNA-Chips), die mit dieser Strategie erzeugt wurden, einen weit größeren Anwendungsbereich, da mit diesen sowohl alle Experimente durchgeführt werden können wie mit den "üblichen" DNA-Chips, aber darüberhinaus noch hochparallel festphasengestützte Enzymreaktionen (z.B. cDNA-Synthese, Ligase-Reaktionen, reverse Transkription, PCR, multiplex-PCR) durchgeführt werden können. Damit erschließen sich neue Anwendungsgebiete (z.B. DNA-Computing, festphasengestützte Sequenzierung).

Die Erfindung wird weiter anhand der nachfolgenden Figuren beschrieben.
- Fig. 1:: Herstellung eines Acylierungsreagenzes (allgemein)
- (a): Herstellung des Acylierungsreagenzes für X = OCO
- (b): Herstellung des Acylierungsreagenzes für X = OCS
- (c): Herstellung des Acylierungsreagenzes für X = SO₂
- Fig. 2:: Allgemeiner Syntheseplan erfindungsgemäßer 3'-photolabiler Nucleosid-Derivate

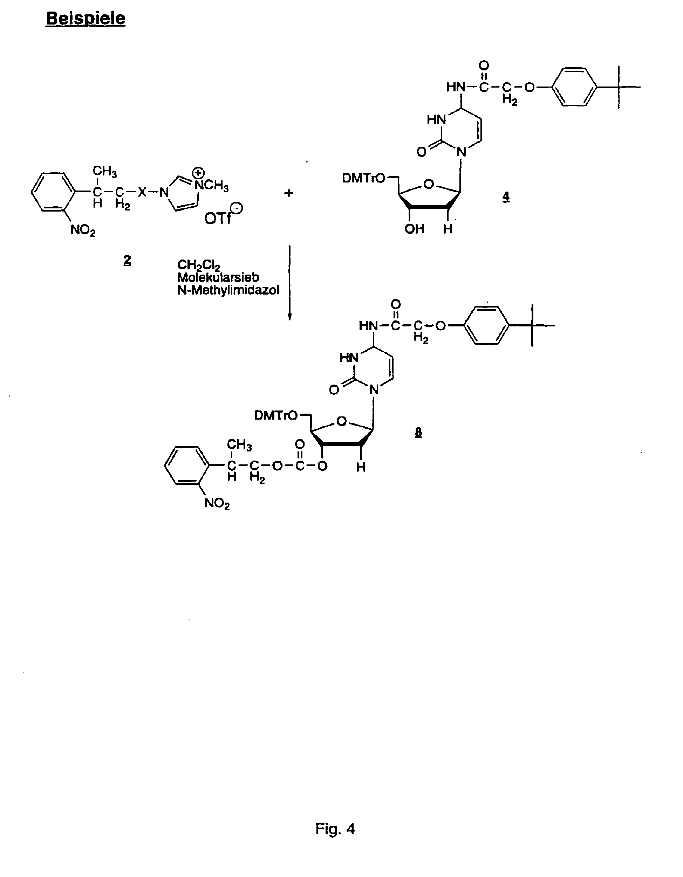

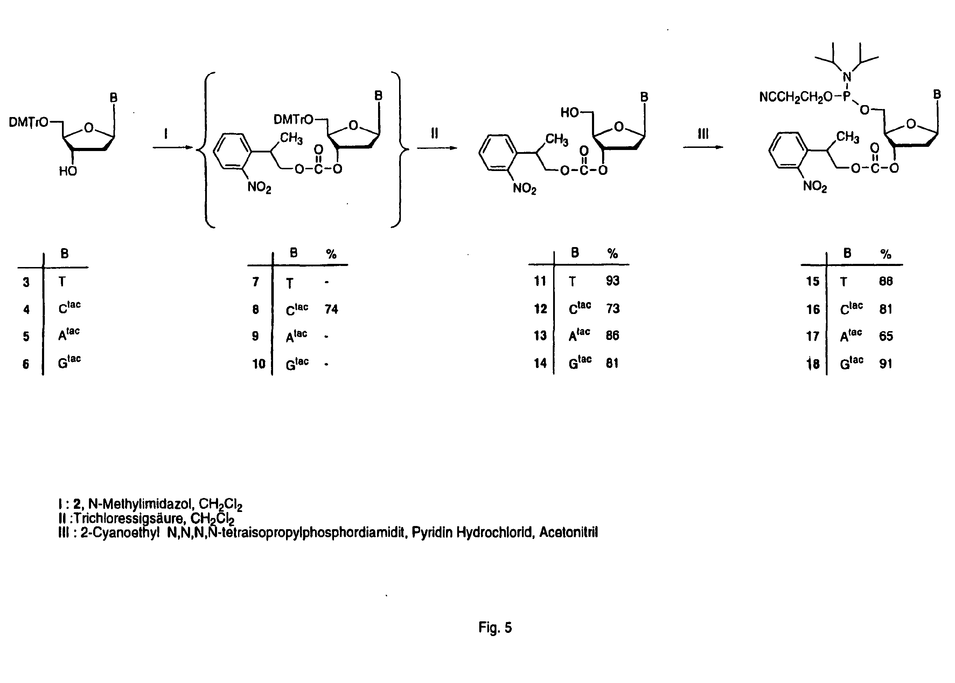

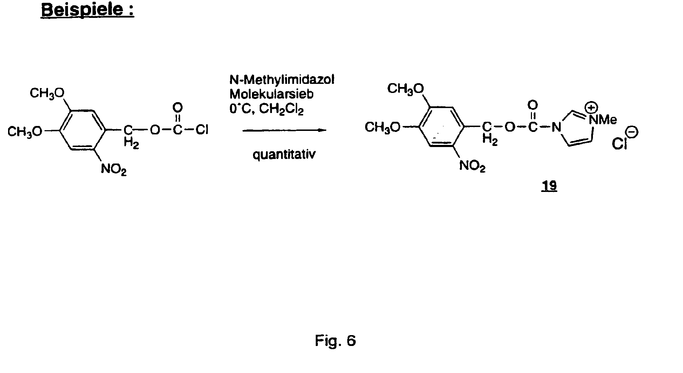

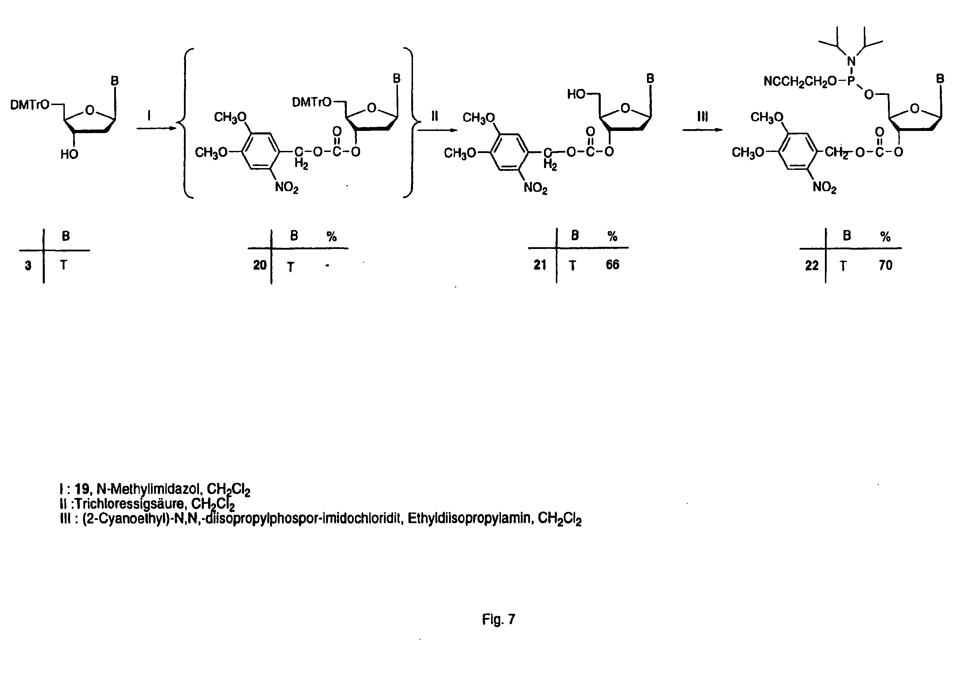

- Fig. 3-7:: Synthesepläne der Verbindungen gemäß der Beispiele 1-16

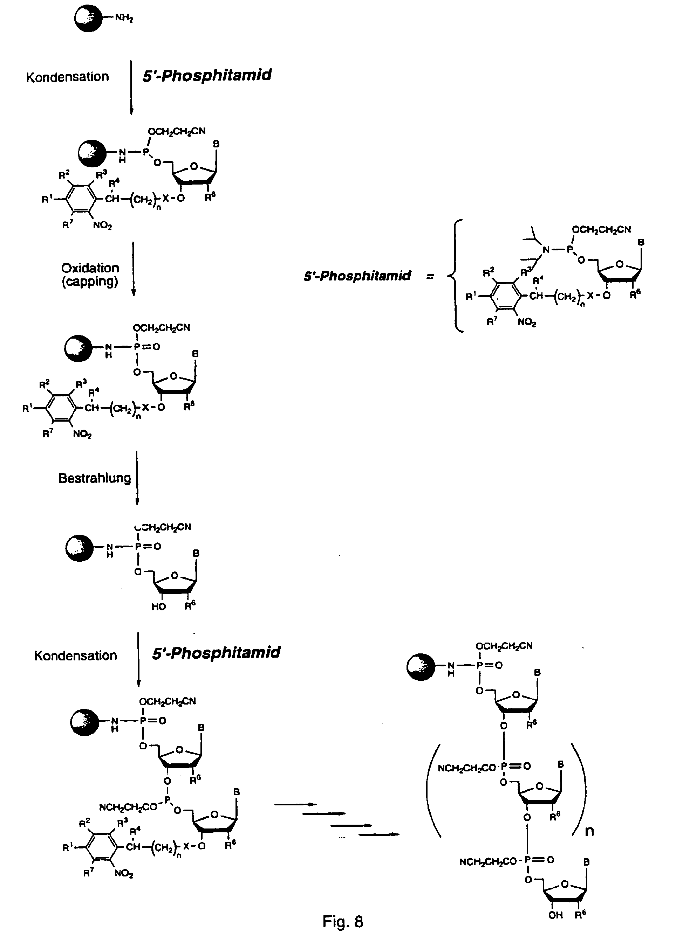

- Fig. 8:: Aufbau eines Oligonukleotids unter Verwendung 3'-O-photolabiler 5'-Phosphitamide gemäß Formel (I)

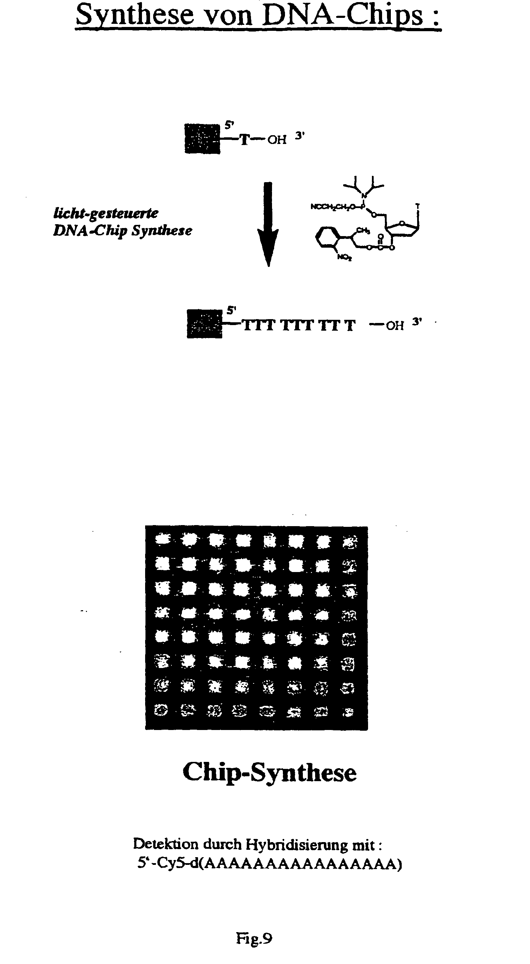

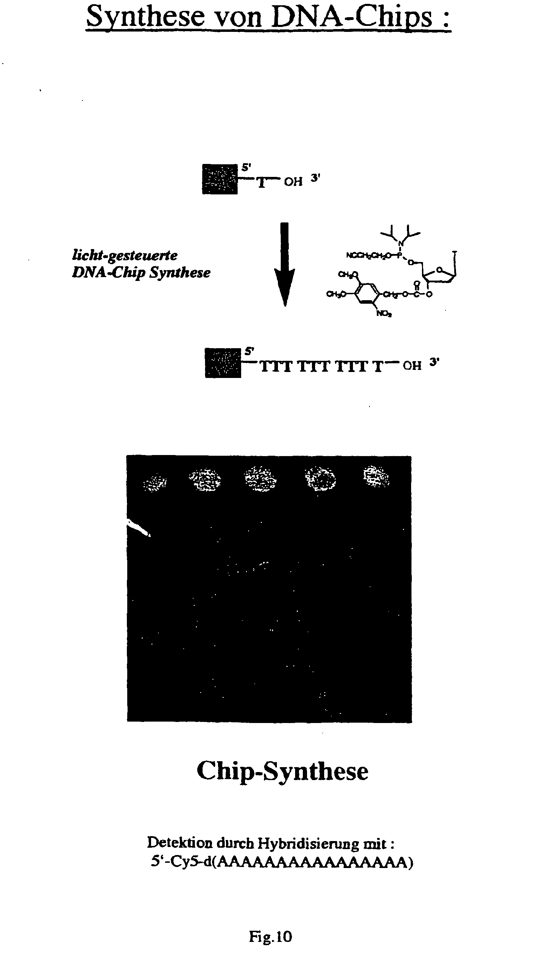

- Fig. 9:: Fluoreszenz-Image eines DNA-Chips, der unter Verwendung von 3'-O-[2-(2-Nitrophenyl)propoxycarbonyl]-thymidin-5'-O-[(2-cyanoethyl-)N,N,-diisopropylphosphoramidit] hergestellt wurde. Auf der Trägeroberfläche wurde die Sequenz dT₉ aufgebaut. Das dT₉ - Oligonukleotid ist mit seinem 5'-Ende auf der Oberfläche verankert, das 3'-OH steht für eine Enzymreaktion frei zur Verfügung. Das abgebildete Fluoreszenz-Image wurde nach Hybridisierung des Chips mit Cy5-markiertem dA₁₆ erhalten. Das Muster entspricht der verwendeten Maske. Fig. 10: Fluoreszenz-Image eines DNA-Chips, der unter Verwendung von 3'-O-[6-Nitroveratryl)oxycarbonyl]-thymidin-5'-O-[(2-cyanoethyl-)N,N,-diisopropylphosphoramidit] hergestellt wurde. Auf der Trägeroberfläche wurde die Sequenz dT₁₀ aufgebaut. Das dT₁₀ - Oligonukleotid ist mit seinem 5'-Ende auf der Oberfläche verankert, das 3'-OH steht für eine Enzymreaktion frei zur Verfügung. Das abgebildete Fluoreszenz-Image wurde nach Hybridisierung des Chips mit Cy5-markiertem dA₁₆ erhalten. Das Muster entspricht der verwendeten Maske.

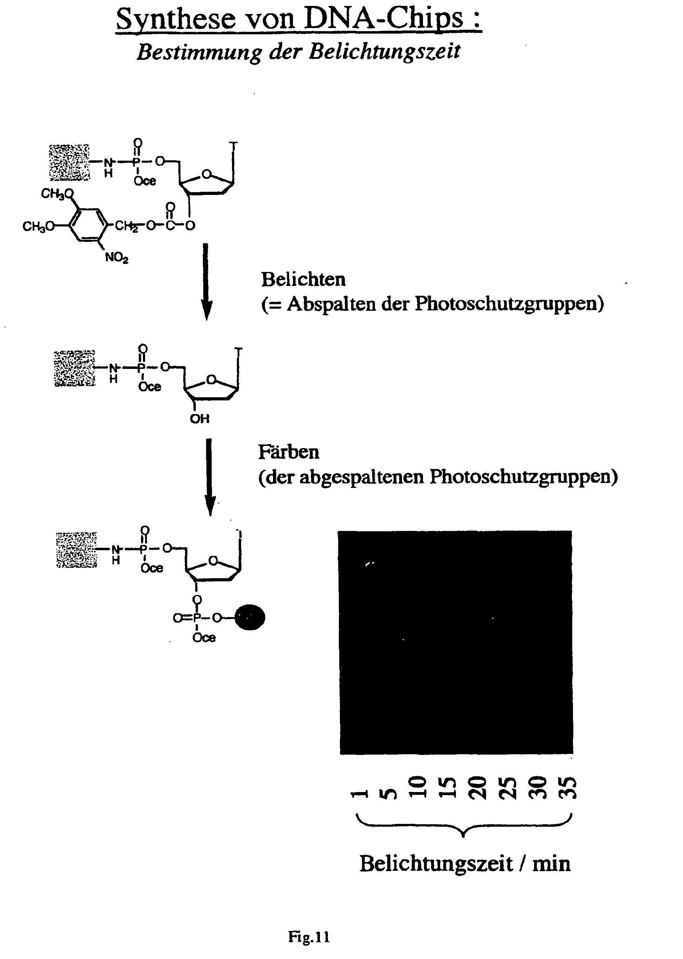

- Fig. 11: zeigt das Fluoreszenz-Image eines DNA-Chips der zur Bestimmung der Bestrahlungszeit für die 3'-O-[(6-Nitroveratryl)oxycarbonyl]-Schutzgruppe verwendet wurde. Von links nach rechts wurde mit 1, 5, 10, 15, 20, 25, 30, 35 min ein oberflächengebundener 3'-O-[(6-Nitroveratryl)oxycarbonyl]-Baustein bestrahlt. Im Anschluss wurde die erfolgte Abstraktion der von 3'-O-[(6-Nitroveratryl)oxycarbonyl]-Gruppen durch permanentes Labeling mit einem Cy5-Farbstoff sichtbar gemacht. Das Muster entspricht der verwendeten Maske.

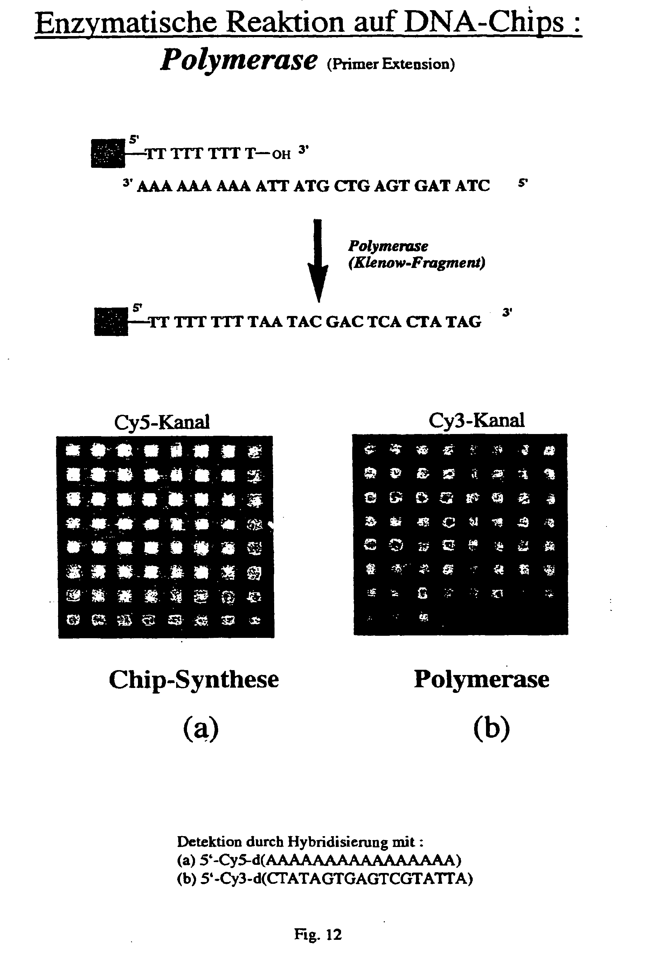

- Fig. 12: Polymerase-Reaktion auf einem DNA-Chip (Sequenz: dT₉), der unter Verwendung von 3'-O-[2-(2-Nitrophenyl)propoxycarbonyl]-thymidin-5'-O-[(2-cyanoethyl-)-N,N-diisopropyl-phosphoramidit] hergestellt wurde. Die Polymerase-Reaktion (Primer Extension) wurde mit den abgebildeten Nucleotid-Sequenzen durchgeführt und deren Erfolg mittels Hybridisierung sichtbar gemacht. Gezeigt sind die Fluoreszenz-Images, die nach gleichzeitiger Hybridisierung mit Cy5-markierten dA₁₆ und Cy3-markiertem d(CTATAGTGAGTCGTA) erhalten wurden. Mit Cy5-dA₁₆ wird die mittels licht-gesteuerter Synthese auf der Trägeroberfläche erzeugte dT₉-Sequenz detektiert; mit Cy3-d(CTATAGTGAGTCGTA) wird ausschließlich die Kettenverlängerung des oberflächengebundenen Primers durch die Polymerase-Reaktion detektiert.

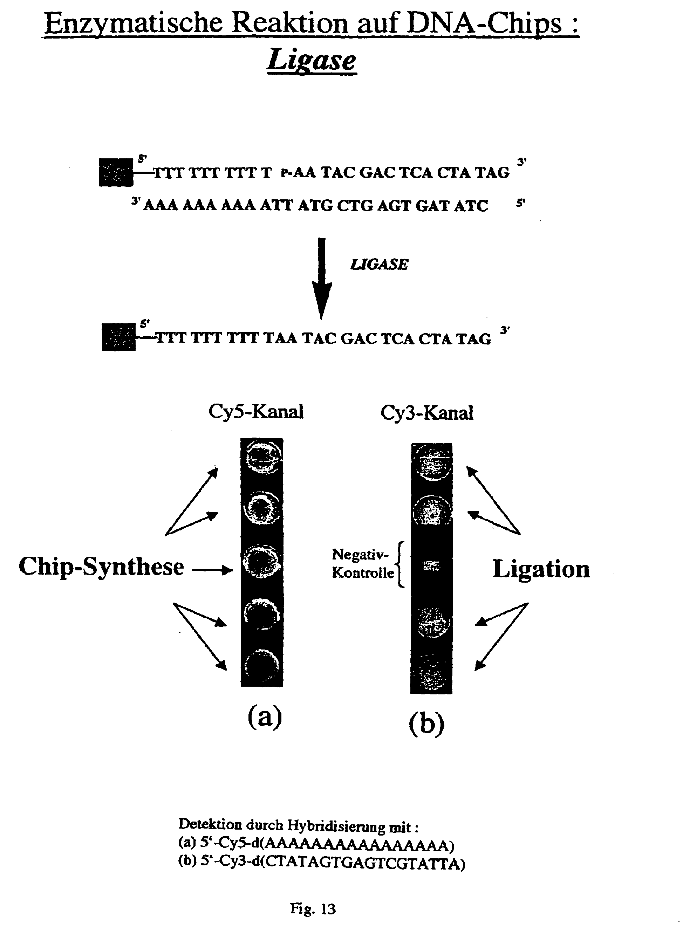

- Fig. 13: Ligase-Reaktion auf einem DNA-Chip (Sequenz: dT₁₀), der unter Verwendung von 3'-O-[2-(2-Nitrophenyl)propoxycarbonyl]-thymidin-5'-O-[(2-cyanoethyl-)-N,N-diisopropylphosphoramidit] hergestellt wurde. Die Ligase-Reaktion wurde mit den abgebildeten Nucleotid-Sequenzen durchgeführt und deren Erfolg mittels Hybridisierung sichtbar gemacht. Gezeigt sind die Fluoreszenz-Images, die nach gleichzeitiger Hybridisierung mit Cy5-markiertem dA₁₆ und Cy3-markiertem d(CTATAGTGAGTCGTA) erhalten wurden. Mit Cy5-dA₁₆ wird die mittels lichtgesteuerter Synthese auf der Trägeroberfläche erzeugte dT₁₀-Sequenz detektiert; mit Cy3-d(CTATAGTGAGTCGTA) wird ausschliesslich die Anknüpfung der Sequenz d(5'-Phosphat-AATACGACTCACTA-TAG) durch die Ligase-Reaktion detektiert. In der Mitte der Arrays wurde als Negativkontrolle keine Ligasereaktion durchgeführt und daher ist dieser Spot auch nur im Cy5-Kanal und nicht im Cy3-Kanal sichtbar.
Die Erfindung wird weiter anhand der nachfolgenden Beispiele beschrieben.

Die Reaktionsschemata zur Herstellung der nachfolgenden Verbindungen ist in den Figuren 3-7 gezeigt, worauf nachfolgend Bezug genommen wird.

**Reagenzien**: DMTr-geschützte Nucleoside, 2-Cyanoethyl- N,N,N,N-tetraisopropyl-phosphoro-diamidite und 2-Cyanoethyl-N,N-diisopropylphosphor-imidochloridit von Proligo (Hamburg, Germany). Alle anderen Reagenzien von Fluka (Ulm, Germany)

### Beispiel 1: 2-(2-Nitrophenyl)propoxycarbonylchlorid (1)

Zu 5 ml Diphosgen (41.4 mmol) in 10 ml absolutem THF werden unter Stickstoffatmosphäre bei 0°C über eine Kanüle eine Lösung bestehend aus 7.2 g 2-(2-Nitrophenyl)propanol (39.7 mmol) und 4.4 ml N-Methylmorpholin (39.7 mmol) in 15 ml absolutem THF langsam zugegeben. Nach 1 hr Rühren bei 0°C wird vom gebildeten Niederschlag abgesaugt und das Filtrat am Hochvakuum abgezogen. Man erhält 6.91g von 1 in Form eines braunen Öls (71 %).

### Beispiel 2: N³-[2-(2-Nitrophenyl)propoxycarbonyl]-N-methyl-imidazoliumchlorid (2)

1.07 ml 2-(2-Nitrophenyl)propoxycarbonylchlorid (1) (4.4 mmol) werden langsam zu einer Lösung von 1.24 ml N-Methylimidazol (14.7 mmol) in 40 ml Dichloromethan über Molsieb 4Å bei 0°C zugetropft. Nach 30 min Rühren im Eisbad wird diese Lösung direkt mit 1.2 Equivalenten der 5'-O-geschützten Nucleosidbausteine zur Acylierung eingesetzt werden.

### Beispiel 3: 1-Methyl-3-[2-(2-nitrophenyl)propoxycarbonyl)-imidazoliumtriflat (2a)

Unter Stickstoffatmosphäre werden 2.19 g N,N-Carbonyldiimidazol (13.5 mmol) in 40 ml absolutem Dichlormethan und 10 ml absolutem Nitromethan gelöst und auf 0°C gekühlt. Es werden 3 ml Trifluormethansulfonsäuremethylester (27 mmol) zugegeben und bei 0°C gerührt. Nach 30 min wird eine Lösung bestehend aus 1.22 g 2-(2-Nitrophenyl)propanol (6.75 mmol) in 10 ml absolutem Dichlormethan zugegeben. Die Reaktionslösung kann nach 1 hr Reaktionszeit direkt mit 1.2 Equivalenten der 5'-O-geschützten Nucleosidbausteine zur Acylierung eingesetzt werden.

### Beispiel 4: N4-((4-^{tert}butylphenoxy)acetyl)-5'-O-(4,4'-dimethoxy-trityl)-3'-O-[2-(2-nitrophenyl)propoxycarbonyl]-2'-deoxycytidin (8)

Zu 1.2 equiv N³-[2-(2-Nitrophenyl)propoxycarbonyl]-N-methylimidazolium chlorid (**2**) (3.7 mmol) in 50 ml Dichlormethan über Molsieb 4Å werden innerhalb 10 min eine Lösung aus 2.27 g N4-((4-^{tert}butylphenoxy)acetyl)-5'-O-(4,4'-dimethoxytrityl)-2'-O-desoxycytidin **(4)** (3.1 mmol) in 20 ml Dichlormethan bei 0°C zugetropft. Das Reaktionsgemisch wurde über Nacht bei 0°C gerührt, dann mit gesättigter NaHCO₃ (100 ml) extrahiert, über Na₂SO₄ getrocknet und evaporiert. Reinigung über Flash Chromatographie (0-66 % Ethylacetat in Toluol) ergab 2.12 g (74%) der Titelverbindung.

¹H-NMR (DMSO) : δ_10.90 (br, NH), 8.04 (2d, H-C(6)), 7.80 (m, 1H *o* zu NO₂), 7.67 (m, 1H *m* zu NO₂, 1H *p* zu NO₂), 7.47 (m, 1H *m* zu NO₂), 7.19-7.34 (m, 9H DMTr, 2Hm zu ^{tert}butyl), 6.99 (2d, H-C(5)), 6.84 (m, 4 H DMTr, 2H *o* zu ^{tert}butyl), 6.07 (m, H-C (1')), 5.10 (m, H-C(3')), 4.77 (s, CH₂O), 4.14-4.35 (m, *OCH₂CH,* H-C(4')), 3.70 (m, 2 OCH₃), 3.51 (m, *CH*CH₃), 3.20-3.30 (m, 2 H-C(5')), 2.51 (m, H-C(2')), 2.31 (m, H-C(2')), 1.28 (d, CH*CH₃*), 1.24 (s, C(CH₃)₃). HRMS (FAB, M+H⁺) berechnet für C₅₂H₅₄N₄O₁₂: 927.3816. Gefunden: 927.3826. ESI-MS: 927 (M+H⁺), 950 (M+Na⁺). R_{f} (Toluol/Ethylacetat 2:1) 0.34

### Beispiel 5: N4-((4-^{tert}butylphenoxy)acetyl)-5'-O-(4,4'-dimethoxy-trityl)-3'-O-[2-(2-nitrophenyl)propoxycarbonyl]-2'-deoxycytidin (8)

Zu 1.2 equiv 1-Methyl-3-[2-(2-nitrophenyl)propoxy-carbonyl]i-midazoliumtriflat **(2a)** (2.8 mmol) in 8 ml Dichlormethan und 2 ml Nitromethan über Molsieb 4Å werden innerhalb 10 min eine Lösung aus 1.68 g N4-((4-^{tert}butylphenoxy)acetyl)-5'-O-(4,4'-dimethoxytrityl)-2'-O-desoxycytidin **(4)** (2.3 mmol) in 10 ml Dichlormethan bei 0°C zugetropft. Das Reaktionsgemisch wurde über Nacht bei 0°C gerührt, dann mit gesättigter NaHCO3 (100 ml) extrahiert, über Na₂SO₄ getrocknet und evaporiert. Die Aufreinigung der Titelverbindung erfolgt über Flash Chromatographie (0-66 % Ethylacetat in Toluol).
R_{f} (Toluol/Ethylacetat 2:1) 0.34

### Beispiel 6: 3'-o-[2-(2-Nitrophenyl)propoxycarbonyl]-thymidin (11)

Zu 1.2 equiv N³-[2-(2-Nitrophenyl)propoxycarbonyl]-N-methylimidazolium chlorid **(2)** (4.4 mmol) in 30 ml Dichlormethan über Molsieb 4Å werden innerhalb 10 min eine Lösung aus 2 g 5'-O-(4,4'-Dimethoxytrityl)-thymidin **(3)** (3.67 mmol) in 20 ml Dichlormethan bei 0°C zugetropft. Das Reaktionsgemisch wurde über Nacht bei 0°C gerührt, dann mit 0.5 % HCl (100 ml) extrahiert, über Na₂SO₄ getrocknet und evaporiert. Zur organischen Phase wird 10 % Trichloressigsäure (70 ml) in Dichlormethan zugefügt und für 2 min gerührt. Danach wird die tief rote Lösung zweimal mit gesättigter NaHCO₃ (100 ml) extrahiert, über Na₂SO₄ getrocknet und evaporiert. Reinigung über Flash Chromatographie (0-10 % Methanol in Toluol/Ethylacetat (5:4)) ergab 1.53 g (93%) der Titelverbindung.

¹H-NMR (DMSO) : δ_11.26 (br, NH), 7.82 (m, 1H *o* zu NO₂), 7.69 (m, H-C (6), 1H *m* zu NO₂, 1H *p* zu NO₂), 7.49 (m, 1H *m* zu NO₂), 6.11 (m, H-C(1')), 5.09 (m, H-C(3'), HO-C(5')), 4.33 (m, CH*CH₂*-O), 3.96 (m, H-C(4')), 3.59 (2m, 2 H-C(5')), 3.52 (m, *CH*CH₂O), 2.24 (m, 2 H-C(24)), 1.77 (2s, CH₃), 1.29 (d, CH*CH₃*). HRMS (FAB, M+H⁺) berechnet für C₂₀H₂₃N₃O₉ : 450.1512. Gefunden: 450.1-524. ESI-MS: 450 (M+H⁺), 472 (M+Na⁺) , 899 (2M+H⁺), 921 (2M+Na⁺). R_{f} (Toluol/Ethylacetat 1:2) 0.21

### Beispiel 7: N4-((4-^{tert}butylphenoxy)acetyl)-3'-O-[2-(2-nitrophenyl)-propoxycarbonyl]-2'-desoxycytidin (12)

Wie beschrieben für 11 mit N4-((4-^{tert}butylphenoxy)acetyl)-2'-desoxycytidin **(4)** (5 g, 6.93 mmol) in 50 ml Dichlormethan und 2 (2.71 g, 8.32 mmol) in 50 ml Dichlormethan. Reinigung durch Ausfällen mit Toluol ergab 3.16 g (73%) der Titelverbindung. ¹H-NMR (DMSO) : δ_10.87 (br, NH), 8.29 (d, H-C(6)), 7.82 (m, 1H o zu NO₂), 7.69 (m, 1H *m* zu NO₂, 1H *p* zu NO₂), 7.48 (m, 1H *m* zu NO₂), 7.29 (m, 2H *m* zu ^{tert}butyl) 7.13 (d, H-C(5)), 6.84 (m, 2H *o* zu ^{tert}butyl), 6.08 (m, H-C (1')) 5.10 (m, H-C(3'), HO-C(5')), 4.77 (s, CH₂O), 4.33 (m, *OCH₂CH*), 4.10 (m, H-C(4')), 3.62 (m, 2 H-C(5')), 3.53 (m, *CH*CH₃), 2.48 (m, H-C(2')), 2.21 (m, H-C(2')), 1.29 (d, CH*CH₃*), 1.24 (s, C(*CH*₃)₃). HRMS (FAB, M+H⁺) berechnet für C₃₁H₃₆N₄O₁₀: 625.2509. Gefunden: 625.2495. ESI-MS: 625 (M+H⁺), 647 (M+Na⁺), 1249 (2M+H⁺), 1271 (2M+Na⁺). R_{f} (Toluol/Ethylacetat 1:4) 0.50

### Beispiel 8: N6-((4-^{tert}butylphenoxy)acetyl)-3'-O-[2-(2-nitrophenyl)-propoxycarbonyl]-2'-desoxyadenosin (13)

Wie beschrieben für 11 mit N4-((4-^{tert}butylphenoxy)acetyl)-2'-desoxyadenosin (5) (2.73 g, 3.67 mmol) in 50 ml Dichlormethan und 2 (1.31 g, 4.40 mmol) in 50 ml Dichlormethan. Reinigung über Flash Chromatographie (0-4 % Methanol in Toluol/Ethylacetat (1:1)) ergab 2.05 g (86%) der Titelverbindung. ¹H-NMR (DMSO) : δ_10.78 (br, NH), 8.66 (m, H-C(2), H-C(8)), 7.83 (m, 1H *o zu* NO₂), 7.70 (m, 1H *m zu* NO₂, 1H *p zu* NO₂), 7.49 (m, 1H *m* zu NO₂), 7.30 (m, 2H *o* zu ^{tert}butyl), 6.89 (m, 2H *m* zu ^{tert}butyl), 6.43 (m, H-C(1')), 5.28 (m, H-C(3')), 5.14 (m, HO-C(5')), 4.98 (s, *CH₂*O), 4.34 (m, *OCH₂*CH), 4.12 (m, H-C(4')), 3.60 (m, 2 H-C(5'), *CH*CH₃), 3.02 (m, H-C(2')), 2.57 (m, H-C (2')), 1.31 (d, CH*CH₃*), 1.24 (s, C(*CH₃*)₃). HRMS (FAB, M+H⁺) berechnet für C₃₂H₃₆N₆O₉: 649.2621. Gefunden: 649.2644. ESI-MS: 649 (M+H⁺), 671 (M+Na⁺), 1297 (2M+H⁺), 1319 (2M+Na⁺)). R_{f} (Toluol/Ethylacetat 1:1) 0.17

### Beispiel 9: N2-((4-^{tert}butylphenoxy)acetyl)-3'-O-(2-(2-nitrophenyl)-propoxycarbonyl]-2'-desoxyguanosin (14)

Wie beschrieben für 11 mit N2-((4-^{tert}butylphenoxy)acetyl)-2'-desoxyguanosin (6) (5 g, 6.58 mmol) in 50 ml Dichlormethan und 2 (2.57 g, 7.9 mmol) in 50 ml Dichlormethan. Reinigung über Flash Chromatographie (0-10 % Methanol in Toluol/Ethylacetat (1:1)) ergab 3.53 g (81%) der Titelverbindung.

¹H-NMR (DMSO) : δ_11.74 (br, 2 NH), 8.23 (2s, H-C(8)), 7.83 (m, 1H *o zu* NO₂), 7.70 (m, 1H *m zu* NO₂, 1H *p zu* NO₂), 7.49 (m, 1H m *zu* NO₂), 7.30 (m, 2H *o* zu ^{tert}butyl), 6.89 (m, 2H *m zu* ^{tert}butyl), 6.18 (m, H-C(1')), 5.13 (m, H-C(3')), 5.06 (m, HO-C(5')-), 4.81 (2s, *CH₂*O), 4.34 (m, *OCH₂*CH), 4.04 (m, H-C(4')), 3.55 (m, 2 H-C(5'), *CH*CH₃), 2.83 (m, H-C(2')), 2.49 (m, H-C(2')), 1.29 (d, CH*CH*₃), 1.25 (s, C(*CH₃*)₃). HRMS (FAB, M+H⁺) berechnet für C₃₂H₃₆N₆O₁₀: 665.2570. Gefunden: 665.2582. ESI-MS: 665 (M+H⁺), 687 (M+Na⁺)), 1329 (2M+H⁺), 1351 (2M+Na⁺). R_{f} (Ethylacetat/Methanol 3:1) 0.18

### Beispiel 10: 3-O-[2-(2-Nitrophenyl)propoxycarbonyl]-thymidin-5'-O-[(2-cyanoethyl-)-N,N-diisopropylphosphoramidit] (15)

Zu einer Lösung aus 1.53 g 3'-O-[2-(2-Nitrophenyl)propoxycarbonyl]-thymidin (11) (3.4 mmol) in 15 ml Acetonitril werden 1.2 ml 2-Cyanoethyl-N,N,N,N-tetraisopropylphosphorodiamidit (3.98 mmol) und 0.5 M Pyridin Hydrochlorid (3.4 ml, 1.7 mmol) in Acetonitril zugefügt. Nach 1 hr Rühren wird die Reaktionslösung mit Dichlormethan (100 ml) verdünnt und mit gesättigter NaHCO₃ (100 ml) extrahiert. Die organische Phase wird mit gesättigter NaCl (100 ml) gewaschen, über Na₂SO₄ getrocknet und evaporiert. Reinigung über Flash Chromatographie (0-30 % Ethylacetat in Toluol) ergab 1.94 g (88%) der Titelverbindung.

¹H-NMR (DMSO) : δ_11.26 (br, NH), 7.82 (m, 1H *o* zu NO₂), 7.69 (m, 1H *m* zu NO₂, 1H *p zu* NO₂), 7.47-7.55 (m, H-C (5), 1H m zu NO₂) , 6.08 (m, H-C(1')), 5.09 (m, H-C(3')), 4.27-4.35 (m, O*CH₂*-CH₂), 4.12 (m, H-C(4')), 3.70-3.83 (m, 2 H-C(5'), OCH₂ CH₂CN), 3.49-3.59 (m, 3 *CH*CH₃), 2.75 (m, CH₂*CH₂*CN), 2.29 (m, 2 H-C(2')), 1.78 (m, CH₃), 1.28 (d, CH₃), 1.09-1.24 (m, 7 CH₃). ³¹P-NMR (DMSO) : δ_149.36, 149.33, 149.29

ESI-MS : 649 (M+H⁺), 672 (M+Na⁺), 1321 (2M+Na⁺). HRMS (FAB, M+H⁺) berechnet für C₂₉H₄₀N₅O₁₀P: 650.2590. Gefunden: 650.2576. R_{f} (Toluol/Ethylacetat 1:1) 0.37

### Beispiel 11: N4-((4-^{tert}butylphenoxy)acetyl)-3'-O-[2-(2-nitrophenyl)-propoxycarbonyl]-2'-desoxycytidin-5'-O-[(2-cyano-ethyl)-N,N-diisopropylphosphoramidit] (16)

wie beschrieben für 15, mit N4-((4-^{tert}butylphenoxy)acetyl)-3'-O-12-(2-nitrophenyl)propoxy-carbonyl]-2'-desoxycytidin (12) (1.51 g, 2.41 mmol), 2-Cyanoethyl- N,N,N,N-tetraisopropylphosphordiamidit (0.9 ml, 2.84 mmol) und 0.5 M Pyridin Hydrochlorid (2.6 ml, 1.3 mmol). Reinigung über Flash Chromatographie (0-30 % Ethylacetat in Toluol) ergab 1.61 g (81%) der Titelverbindung.

¹H-NMR (DMSO) : δ_10.90 (br, NH), 8.14 (m, H-C(6)), 7.82 (m, 1H o zu NO₂), 7.69 (m, 1H *m zu* NO₂, 1H *p* zu NO₂, 7.48 (m, 1H *m* zu NO₂), 7.29 (m, 2H *o zu* ^{tert}butyl), 7.14 (m, H-C(5)), 6.83 (m, 2H *m zu* ^{tert}butyl), 6.07 (m, H-C(1')), 5.10 (m, H-C(3')), 4.77 (s, OCH₂), 4.30 (m, OCH₂CH₂, H-C(4')), 3.75 (m, 2 H-C(5'), O*CH₂* CH₂CN), 3.55 (m, 3 CHCH₃), 2.73 (m, CH₂*CH₂*CN), 2.55 (m, H-C(2')-), 2.25 (m, H-C(2')), 1.29 (m, CH₃), 1.15 (m, 7 CH₃). ³¹P-NMR (DMSO) : δ_149.35. HRMS (FAB, M+H⁺) berechnet für C₄₀H₅₃N₆O₁₁P: 825.3587. Gefunden: 825.3568. ESI-MS: 825 (M+H⁺), 847 (M+Na⁺), 1649 (2M+H⁺), 1671 (2M+Na⁺). R_{f} (Poluol/Ethylacetat 1:1) 0.43

### Beispiel 12: N6-((4-^{tert}butylphenoxy)acetyl)-3'-O-[2-(2-nitrophenyl)-propoxycarbonyl]-2'-dssoxyadenosin-5'-O-[(2-cyano-ethyl)-N,N-diisopropylphosphoramidit] (17)

Wie beschrieben für 15, mit N6-((4-^{tert}butylphenoxy)acetyl)-3'-O-[2-(2-nitro-phenyl)propoxycarbonyl]-2'-desoxyadenosin (13) (1.90 g, 2.93 mmol), 2-Cyanoethyl- N,N,N,N-tetraisopropylphosphordiamidit (1.2 ml, 3.78 mmol) und 0.5 M Pyridin Hydrochlorid (2.9 ml, 1.45 mmol). Reinigung über Flash Chromatographie (0-30 % Ethylacetat in Toluol) ergab 1.9 g (65%) der Titelverbindung.

¹H-NMR (DMSO) : δ_11.70 (br, NH), 8.64 (m, H-C(2), H-C(8)), 7.83 (m, 1H *o zu* NO₂), 7.71 (m, 1H *m zu* NO₂, 1H *p zu* NO₂, 7.48 (m, 1H *m zu* NO₂), 7.29 (m, 2H *o zu* ^{tert}butyl), 6.88 (m, 2H m zu ^{tert}butyl), 6.45 (m, H-C(1')), 5.33 (m, H-C(3')), 4.98 (s, OCH₂), 4.36 (m, OCH₂CH₂), 4.24 (m, H-C(4')), 4.78 (m, 2 H-C(5'), *OCH₂* CH₂CN), 3.53 (m, 3 CHCH₃), 3.12 (m, H-C(2')), 2.74 (m, CH₂*CH₂*CN-), 2.60 (m, H-C(2')), 1.30 (d, CH₃), 1.11 (m, 7 CH₃). ³¹P-NMR (DMSO) : δ_149.24, 149.20, 149.16. HRMS (FAB, M+H⁺) berechnet für C₄₁H₅₃N₈O₁₀P: 849.3700. Gefunden: 849.3723. ESI-MS: 849 (M+H⁺-), 871 (M+Na⁺), 1697 (2M+H⁺), 1719 (2M+Na⁺). R_{f} (Toluol/Ethylacetat 1:1) 0.53

### Beispiel 13: N2-((4-^{tert}butylphenoxy)acetyl)-3'-O-[2-(2-nitrophenyl)-propoxycarbonyl]-2'-desoxyguanosin-5'-O-[(2-cyano-ethyl)-N,N-diisopropylphosphoramidit] (18)

Wie beschrieben für 15, mit N2-((4-^{tert}butylphenoxy)acetyl)-3'-*o*-[2-(2-nitrophenyl)propoxy-carbonyl]-2'-desoxyguanosin (14) (1.0 g, 1.50 mmol), 2-Cyanoethyl- N,N,N,N-tetraisopropylphosphordiamidit (0.9 ml, 2.84 mmol) und 0.5 M Pyridin Hydrochlorid (1.75 ml, 0.87 mmol). Reinigung über Flash Chromatographie (33-66 % Aceton in Petrolether) ergab 1.19 g (91%) der Titelverbindung.

¹H-NMR (DMSO) : δ_11.45 (br, 2 NH), 8.15 (m, H-C (8)), 7.83 (m, 1H *o zu* NO₂), 7.70 (m, 1H *m* zu NO₂, 1H *p zu* NO₂, 7.49 (m, 1H *m* zu NO₂ ), 7.29 (m, 2H *o zu* ^{tert}butyl), 6.88 (m, 2H *m* zu ^{tert}butyl), 6.19 (m, H-C(1')), 5.23 (m, H-C(3')), 4.79 (m, *OCH₂),* 4.36 (m, OCH₂CH₂), 4.20 (m, H-C(4')), 3.73 (m, 2 H-C(5'), *OCH₂* CH₂CN), 3.55 (m, 3 *CH*CH₃), 2.87 (m, CH₂*CH₂*CN), 2.76 (m, H-C(2')-), 2.57 (m, H-C(2')), 1.30 (d, CH₃), 1.15 (m, 7 CH₃). ³¹P-NMR (DMSO) : δ_149.46, 149.41. HRMS (FAB, M+H⁺) berechnet für C₄₁H₅₃N₈O₁₁P: 865.3649. Gefunden: 865.3660. ESI-MS: 865 (M+H⁺), 887 (M+Na⁺), 1751 (2M+Na⁺). R_{f} (Toluol/Ethylacetat/Methanol 5:4:1) 0.39

### Beispiel 14: N-Methyl-N3-[(6-nitroveratryl)oxycarbonyl]-imidazoliumchlorid (19)

Bei 0°C werden zu 1.44 ml N-Methylimidazol (18.1 mmol) und Molekularsieb 4Å in 100 ml absolutem Dichlormethan 2 g Chlorameisensäure-6-nitroveratrylester (7.25 mmol; Firma Fluka Ulm) zugegeben. Die Reaktionslösung wird 15 Minuten bei 0°C gerührt. Diese Reaktionslösung wird direkt für Acylierungen eingesetzt.

### Beispiel 15: 3'-O-[(6-Nitroveratryl)oxycarbonyl]-thymidin (21)

Eine Lösung von 1.97 g 5'-O-(4,4'-Dimethoxytrityl)-thymidin (3.62 mmol) in 18 ml absolutem Dichlormethan und und 2 ml absolutem Pyridin werden unter Stickstoffatmosphäre und unter Ausschluss von Licht zu einer N-Methyl-N3-[(6-nitroveratryl)oxycarbonyl]-imidazoliumchlorid-Acylierungsreaktion (19) (hergestellt aus 7.25 mmol Chlorameisensäure-6-nitroveratrylester) zugefügt. Das Reaktionsgemisch wird abgedunkelt über Nacht bei 4°C gerührt. Das Molekularsieb wird abgetrennt und die organische Phase zweimal gegen gesättigte NaHCO₃ (100 ml) extrahiert. Nach Trockenen über Na₂SO₄ werden der organischen Phase 200 ml einer 10 % Trichloressigsäure in Dichlorethan zugefügt und 5 min bei Raumtemperatur gerührt. Die stark rot gefärbte Lösung wird zweimal mit gesättigter NaHCO₃ (200 ml) extrahiert, die organische Phase über Na₂SO₄ getrocknet und evaporiert. Reinigung über Flash Chromatographie (66-80% Ethylacetat in Toluol) ergab 1.153 g der Titelverbindung (66 % Ausbeute)

### Beispiel 16: 3'-O-[(6-Nitroveratryl)oxycarbonyl]-thymidin-5'-O-[(2-cyanoethyl)-N,N-diisopropylphosphoramidit] (22)

Unter Stickstoffatmosphäre werden 1g 3'-O-[(6-Nitroveratryl)oxycarbonyl]-thymidin **(21)** (2.08 mmol) in 20 ml absolutem Dichlormethan gelöst. Es werden unter Stickstoffatmosphäre und unter Ausschluss von Licht 0.22 ml N-Methylmorpholin (2 mmol) und 0.24 ml 2-Cyanoethyl-N,N-diisopropylphosphor-imidochloridit (1.1 mmol) zugefügt. Nach 1 hr Rühren wird gegen gesättigte NaHCO₃ (200 ml), dann gegen gesättigte NaCl (200 ml) extrahiert, die organische Phase über Na₂SO₄ getrocknet und evaporiert. Reinigung über Flash Chromatographie (50-66% Ethylacetat in Toluol), ergab 0.74 g der Titelverbindung (55 % Ausbeute).

### Beispiel 17: Herstellung von DNA-Chips mit Hilfe von monomeren Bausteinen vom Typ 3'-O-[2-(2-nitrophenyl)propoxycarbonyl]-5'-Bhosphoramidit

Die DNA-Chip-Synthese wurde analog dem Verfahren von Fodor et al. (Science 1991, 251, S 767 ff) unter Verwendung von Masken bzw. maskenfrei, wie bereits in den deutschen Patentanmeldungen DE 198 58 WO 2000; 35931 A2), bzw. DE 199 62 803.3 WO 2001/47627 A1, gezeigt, auf einer Glasoberfläche als Träger durchgeführt.

Die Bestrahlung zum Zwecke der Abstraktion der 3'-O-[2-(2-Nitrophenyl)propoxycarbonyl]-Gruppe wird zwecimäßig unter Zusatz einer Base während der Bestrahlung durchgeführt. Hierbei sei auf die deutsche Patentanmeldung DE 198 58 440.7 verwiesen. Der Reaktionsablauf ist schematisch in Fig. 8 gezeigt. Als monomerer Bausteine wurde die erfindungsgemäße Verbindung 3'-O-[(2-(2-Nitrophenyl)propoxycarbonyl]-thymidin-5'-O[(-cyanoethyl)-N,N-diisopropyl-phosporamidit] eingesetzt. Hierbei sei auf die deutsche Patentanmeldung DE 199 15 867.3 (WO 2000/61594 A2) verwiesen. Der angewendete Zyklus und die speziellen Synthesebedingungen sind in der deutschen Patentanmeldung DE 198 58 440.7 gezeigt, worauf hier Bezug genommen wird. Die Ankoppelung des Nucleotidstranges erfolgt über das 5'-Ende an die feste Trägerphase. Somit ist nach beendeter Synthese und abschließender Schutzgruppenabspaltung des 3'-OH-Ende frei verfügbar. Dadurch können Enzymreaktionen, die ein freies 3'-Ende verlangen (Polymerase-Reaktionen, Ligase-Reaktionen, PCR, cDNA-Synthese, Sequenzierungen, ...) an diesen Bio-Chips durchgeführt werden.

In Fig. 9 ist das Fluoreszenz-Image des hergestellten Chips mit d(T₉) nach Hybridisierung mit Cy5-markiertem d(A₁₆) zu sehen. Das Muster entspricht der angewendeten Maske, d.h. es war eine erfolgreiche DNA-Chip-Synthese möglich.

### Beispiel 18: Herstellung von DNA-Chips mit Hilfe von monomeren Bausteinen vom Typ 3'-O-[(6-Nitroveratryl)oxycarbonyl-5'-Phosporamidit

Die DNA-Chip-Synthese wurde analog dem Verfahren von Fodor et al. (Science 1991, 251, S767 ff) unter Verwendung von Masken bzw. maskenfrei, wie bereits in den deutschen Patentanmeldungen DE 198 58 440.7 bzw. DE 199 62 803.3 gezeigt, auf einer Glas-oberfläche als Träger durchgeführt.

Die Bestrahlung zum Zwecke der Abstraktion der 3'-O-(6-Nitroveratryl)-Gruppe wird zweckmäßig ohne Zusatz von Lösungsmitteln, also "trocken" durchgeführt. Als monomerer Baustein wurde die erfindungsgemäße Verbindung 3'-O-[(6-Nitroveratryl)oxycarbonyl]-thymidin-5'-O-[(2-cyanoethyl)-N,N-disiopropylphosporamidit eingesetzt. Hierbei sei auf die deutsche Patentanmeldung DE 100 03 631. (WO 2000/61594 A3) verweisen, worauf hier Bezug genommen wird. Die Ankoppelung des Nucleotidstranges erfolgt über das 5' -Ende an die feste Trägerphase. Somit ist nach beendeter Synthese und abschließender Schutzgruppenabspaltung das 3'-OH-Ende frei verfügbar. Dadurch können Enzymreaktionen, die ein freies 3'-Ende verlangen (Polymerase-Reaktionen, Ligase-Reaktionen, PCR, cDNA-Synthese, Sequenzierungen, ...) an diesen Bio-Chips durchgeführt werden.

In Fig. 10 ist das Fluoreszenz-Image des hergestellen Chips mit d(T₁₀) nach Hybridisierung mit Cy5-markiertem d(A₁₆) zu sehen. Das Muster entspricht der angewendeten Maske, d.h. es war eine erfolgreiche DNA-Chip-Synthese möglich.

### Beispiel 19: Durchführung einer Polymerase-Reaktion (Primer Extension) auf einem DNA-Chip, der mit Hilfe monomerer Bausteine vom Typ 3'-O-[2-(2-Nitrophenyl)propoxycarbonyl]-thymidin-5'-O-[(2-cyanoethyl-)-N,N-diisopropyl-phosphoramidit hergestellt wurde

Auf den dT₉-DNA-Chip wird für die Durchführung der Enzym-Reaktion eine 25 µl Reaktionskammer (EasiSeal, Hybaid) über den Array fixiert.

1 µl des Templats [d(CTATAGTGAGTCGTATTAAAAAAAAAA), 100 µM] werden in 9,5 µl autoklaviertem Wasser für 5 min bei 95°C denaturiert und nach 3 min auf Eis in die Reaktionskammer gefüllt. Zugegeben werden ferner 12,5 µl des Puffers (20 mM TrisHCl pH 7,5, 10 mM MgCL₂, 15 mM DTT), 1 µl dNTP-Mix (10 mM) und zum Schluß 1 µl des Klenow-Fragments (3'exo+5'exo; 5000 U/ml; New England Biolabs). Nach sorgfältigem Mischen wird die Reaktionskammer verschlossen und die Reaktion über Nacht bei 37°C durchgeführt.

Zur Detektion wird anschließend für 30 sec bei 95°C mit Stripping-Buffer (2,5 mM Na₂HPO₄, 0,1 % (v/v) SDS) gewaschen und dann mit 5'-Cy5-dA16 und 5'-Cy3-d(CTATAGTGAGTCGTATT) hybridisiert.

### Beispiel 20: Durchführung einer Ligase-Reaktion auf einem DNA-Chip, der mit Hilfe monomerer Bausteine vom Typ 3'-O-[2-(2-Nitrophenyl)propoxycarbonyl]-thymidin-5'-O-[(2-eyanoethyl-)-N,N-diisopropylphosphoramidit] hergestellt wurde

Auf den dT₁₀-DNA-Schip wird für die Durchführung der EnzymReaktion eine 25 µl Reaktionskammer (EasiSeal, Hybaid) über den Array fixiert.

1 µl des Templats [d(CTATAGTGAGTCGTATTAAAAAAAAAA), 100 µM werden in 17,8 µl autoklaviertem Wasser in die Reaktionskammer eingefüllt. Nach 2 min erfolgt die Zugabe von 1 µl d(5'-Phosphat-AATACGACTCACTATAG) [100 µM]. Zugegeben werden nach weiteren 2 min ferner 5 µl des Ligase-Puffers (5x; 250 mM TrisHCl pH 7,5, 50 mM MgCl₂, 50 mM DTT, 5 mM ATP, 125 µg BSA/ml) und zum Schluß 0,2 µl der T4-DNA-Ligase (400 00 U/ml; New England Biolbas). die Reaktionskammer wird verschlossen und die Reaktion für 1 hr bei 16°C durchgeführt.

Zur Detektion wird anschließend für 30 sec bei 95°C mit Stripping-Buffer (2,5 mM Na₂HPO₄, 0,1 % (v/v) SDS) gewaschen und dann mit 5'-Cy5-dA16 und 5'-Cy3-d(CTATAGTGAGTCGTATT) hybridisiert.

## Patentansprüche

1. Nucleosid-Derivate mit photolabilen Schutzgruppen der allgemeinen Formel (I) 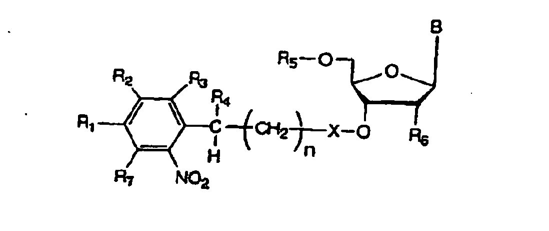 mit
R¹ = H, NO₂, CN, OCH₃, Halogen, Alkyl-, Alkoxy- oder Alkoxyalkylrest mit 1 bis 4 C-Atomen oder ein ggf. substituierter Arylrest oder ein aliphatischer Acylrest mit 2 bis 5 Atomen
R² = H, NO₂, CN, OCH₃, Halogen, Alkyl-, Alkoxy- oder Alkoxyalkylrest mit 1 bis 4 C-Atomen oder ein ggf. substutierter Arylrest oder ein aliphatischer Acylrest mit 2 bis 5 Atomen,
R³ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁴ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁵ = H, Dimethoxytrityl (DMTr) oder eine in der Nucleotidchemie übliche Schutzgruppe oder eine übliche Schutzgruppe zur Herstellung von Oligonukleotiden.
R⁶ = H, OH, Halogen oder ΨR⁸, wobei Ψ = O oder S und R⁸ = Alkyl- oder
Alkoxyalkyl mit 1 bis 4 C-Atomen oder ein ggf. substituierter Arylrest oder ein aliphatischer Acylrest mit 2 bis 5 Atomen sowie eine in der Nukleotidchemie übliche Schutzgruppe
R⁷ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
X= SO₂, OC(O) oder OC(S)
n = 0 oder 1 für X = SO₂
n = 1 für X = OC(O) oder OC(S)
B = H, Adenin, Cytosin, Guanin, Thymin, Uracil, 2,6-Diaminopurin-9-yl,
Hypoxanthin-9-yl, 5-Methylcytosin-1-yl, 5-Amino-4-Imidazolcarbonsäure-1-yl oder 5-Amino-4-Imidazolcarbonsäureamid-3-yl, wobei im Falle von B = Adenin, Cytosin oder Guanin die primäre Aminofunktion ggf. eine temporäre oder permanente Schutzgruppe aufweist bzw. Thymin oder Uracil an der O4-Position ggf. eine permanente Schutzgruppe aufweist.

2. Nucleosid-Derivat nach Anspruch 1, wobei im Falle von R⁴ ≠ H R¹, R² und R³ jeweils H sind.

3. Nucleosid-Derivat nach Anspruch 1, wobei im Fall von R² = OCH₃ R³ = H ist.

4. Nucleosid-Derivat nach einem der Ansprüche 1-3, wobei R⁴ = CH₃ ist.

5. Nucleosid-Derivat nach einem der Ansprüche 1 bis 4, wobei die übliche funktionelle Gruppe zur Herstellung von Oligonukleotiden an der Position R⁵ eine Phosphitamidgruppe der Formel
NC-CH₂-CH₂-O-P-N(Q)₂, p-NC-C₆H₄-CH₂-CH₂-O-P-N(Q)₂, p-NO₂-C₆H₄-CH₂-CH₂-O-P-N(Q)₂ oder CH₂=CH-CH₂-O-P-N(Q)₂ ist,
wobei die Q-Gruppen gleich oder verschieden sein können und lineare oder verzweigte Alkylreste mit 1 bis 4 C-Atomen bedeuten.

6. Nucleosid-Derivat nach Anspruch 5, wobei die Q-Gruppe Ethyl- oder Isopropyl- ist.

7. Nucleosid-Derivat nach einem der Ansprüche 1-6, wobei der Rest R⁸ in der Gruppe ΨR⁸ an der Position R⁸ im Falle von Ψ = 0 eine O-Alkyl-, O-Alkenyl-, O-Acetal- oder O-Silylether-Gruppe oder im Fall von Ψ = S eine O-AlkylGruppe ist.

8. Nucleosid-Derivat nach Anspruch 1, wobei Halogen Cl, Br, I bedeutet.

9. Verfahren zur Herstellung von Nucleosid-Derivaten nach einem der Ansprüche 1-8, wobei man
(a1) eine Verbindung der allgemeinen Formel (II) 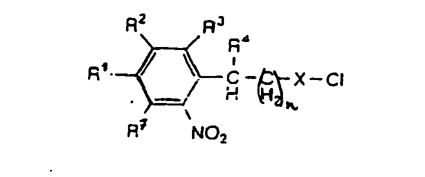 in der R¹, R², R³, R⁴, R⁷, n und X die oben genannte Bedeutung haben,
mit N-Methylimidazol, Pyridin, N,N-Dimethylaminopyridin, Triazol oder Tetrazol umsetzt, oder
(a2) eine Verbindung der allgemeinen Formel (V) 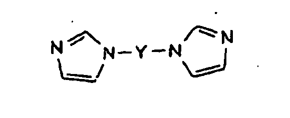 Y = C = 0 oder C = S
mit einem Methylierungsmittel umsetzt sowie das erhaltene Produkt mit einem Alkohol reagieren läßt
und anschließend
(b) das in Stufe (a1) oder (a2) gebildete Acylierungsreagenz (IV) 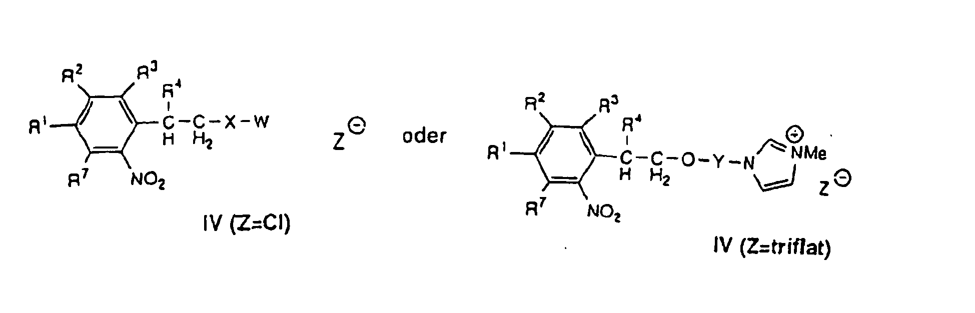 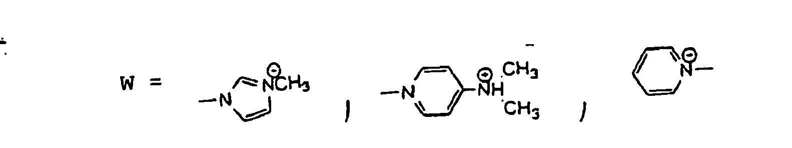 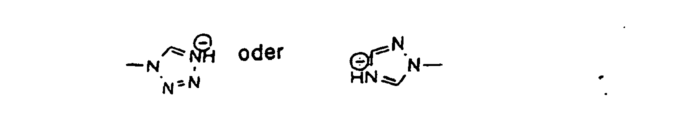 Y = C=O oder C=S
mit einem Nucleosid der allgemeinen Formel (IX) 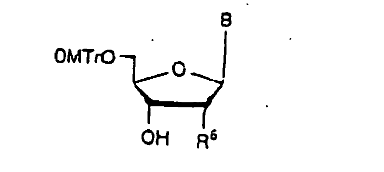 in der R⁵, R⁶ und B die oben angebene Bedeutung haben, reagieren läßt.

10. Verfahren nach Anspruch 9, wobei man in der 5'-Stellung der entstandenen Nucleosid-Derivate eine Phosphitamid-Gruppe der Formel -NC-CH₂-CH₂-O-P-N(Q)₂, p-NC-C₆H₄-CH₂-CH₂-O-P-N (Q) ₂, p-NO₂-C₆H₄-CH₂-CH₂-O-P-N(Q)₂ oder CH₂=CH-CH₂-O-P-N(a)₂ ist,
wobei die Q-Gruppen gleich oder verschieden sein können und lineare oder verzweigte Alkylreste mit 1 bis 4 C-Atomen bedeuten, einführt.

11. Verfahren nach Anspruch 9 oder 10, wobei man Stufe (a1) oder (a2) in einem polaren organischen Lösungsmittel bei Temperaturen zwischen -10 und + 10 °C durchführt.

12. Verfahren nach Anspruch 11, wobei das polare Lösungsmittel Dichlormethan ist.

13. Verfahren nach Anspruch 11 oder 12, wobei die Temperatur ca. 0°C beträgt.

14. Verfahren nach einem der Ansprüche 9 bis 13, wobei man Stufe (b) in Dichlormethan oder einem Dichlormethan enthaltenden Lösungsmittelgemisch bei Temperaturen von ca. 0°C durchführt.

15. Verfahren nach einem der Ansprüche 9 bis 14, wobei man in Stufe (b) das in Stufe (a) gebildete Acylierungsmittel in Dichlormethan vorlegt und das Nucleosid zutropft.

16. Verwendung eines Nucleosid-Derivats mit photolabilen Schutzgruppen der allgemeinen Formel (I) 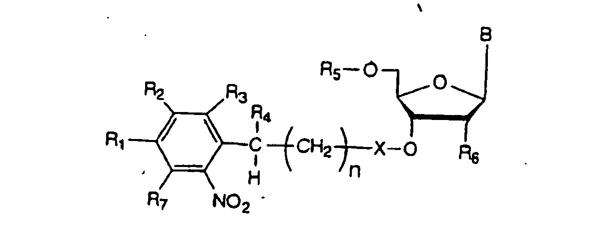 mit
R¹ = H, NO₂, CN, OCH₃, Halogen, Alkyl-, Alkoxy- oder Alkoxyälkylrest mit 1 bis 4 C-Atomen oder ein ggf. substituierter Arylrest oder ein aliphatischer Acylrest mit 2 bis 5 Atomen
R² = H, NO₂, CN, OCH₃, Halogen, Alkyl-, Alkoxy- oder Alkoxyalkylrest mit 1 bis 4 C-Atomen oder ein ggf. substutierter Arylrest oder ein aliphatischer Acylrest mit 2 bis 5 Atomen, R³ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁴ =H, Halogen, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
R⁵ = H, Dimethoxytrityl oder eine in der Nucleotidchemie übliche Schutzgruppe oder eine übliche Schutzgruppe zur Herstellung von Oligonukleotiden
R⁶ = H, OH, Halogen oder ΨR⁸, wobei Ψ = O oder S und R⁸ = Alkyl- oder
Alkoxyalkyl mit 1 bis 4 C-Atomen oder ein ggf. substituierter Arylrest oder ein aliphatischer Acylrest mit 2 bis 5 Atomen sowie eine in der Nukleotidchemie übliche Schutzgruppe
R⁷ = H, NO₂, CN, OCH₃, Halogen, Alkyl-, Alkoxy- oder Alkoxyalkylrest mit 1 bis 4 C-Atomen oder ein ggf. substituierter Arylrest oder aliphatischer Acylrest mit 2 bis 5 Atomen,
n = 0 oder 1
X = SO₂, OC(O), OC(S)
B = H, Adenin, Cytosin, Guanin, Thymin, Uracil, 2,6-Diaminopurin-9-yl. Hypoxanthin-9-yl, 5-Methylcytosin-1-yl, 5-Amino-4-Imidazolcarbonsäure-1-yl oder 5-Amino-4-Imidazolcarbonsäureamid-3-yl, wobei im Falle von B = Adenin, Cytosin oder Guanin die primäre Aminofunktion ggf. eine temporäre oder permanente Schutzgruppe aufweist bzw. Thymin oder Uracil an der O4-Position ggf. eine permanente Schutzgruppe aufweist,
zum Aufbau von Oligonukleotiden oder Nukleinsäure-Chips.

17. Nukleinsäure-Chip, hergestellt unter Verwendung eines Nucleosid-Derivats gemäß Anspruch 16, bei dem die über eine lichtgesteuerte Synthese aufgebauten Oligomeren über das 5'-Ende an die feste Phase gekoppelt sind.

18. Verwendung eines Nukleinsäure-Chips gemäß Anspruch 17 für Enzymreaktionen, die von einer freien 3'-Hydroxylgruppe ausgehen.

19. Verwendung eines Nukleinsäure-Chips gemäß Anspruch 17 für Festphasengestützte Polymerasereaktionen oder Sequenzierung.

20. Verwendung eines Nukleinsäure-Chips gemäß Anspruch 17 für Ligasereaktionen.

21. Verwendung eines Nukleinsäure-Chips gemäß Anspruch 17 für die cDNA-Synthese.

22. Verwendung eines Nukleinsäure-Chips gemäß Anspruch 17 für die reverse Transkription.

23. Verwendung eines Nukleinsäure-Chips gemäß Anspruch 17 für die PCR oder Multiplex-PCR.

24. Verwendung eines Nukleinsäure-Chips gemäß Anspruch 17 für DNA-Computing.

## Claims

1. Nucleoside derivatives having photo-labile protective groups of the general
formula (I) 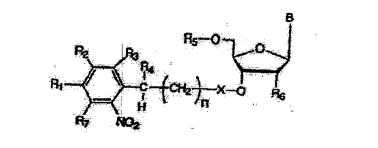 having
R¹ = H, NO₂, CN, OCH₃, halogen, alkyl, alkoxy, or alkoxyalkyl residue
having 1 to 4 C-atoms, or an aryl, optionally substituted, or an aliphatic acyl having 2 to 5 atoms;
R² = = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R³ = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R⁴ = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R⁵ = H, dimethoxytrityl (DMTr), or a protective group common in nucleotide chemistry, or a protective group common for preparing oligonucleotides;
R⁶ = H, OH, halogen or ΨR⁸, wherein Ψ = O or S and R⁸ = alkyl or alkoxyalkyl residue having 1 to 4 C-atoms, or an aryl, optionally substituted, or an aliphatic acyl having 2 to 5 atoms, and a protective group common in nucleotide chemistry R⁷ = H, NO₂, CN, OCH₃, halogen, alkyl, alkoxy, or alkoxyalkyl residue
having 1 to 4 C-atoms, or an aryl, optionally substituted, or an aliphatic acyl having 2 to 5 atoms;
X = = SO₂, OC(O) or OC(S);
n = 0 or 1 for X = SO₂;
n = 1 for X = OC(O) or OC(S);
B - = H, adenine, cytosine, guanine, thymine, uracil, 2.6-diaminopurine-9-yl,
hypoxanthine-9-yl, 5-methylcytosine-1-yl, 5-amino-4-imidazole carboxylic acid-1-yl, or 5-amino-4-imidazole carboxylic acid amide-3-yl, wherein, when B = adenine, cytosine or guanine, the primary amino function has optionally a temporary or permanent protective group, or thymine or uracil have optionally a permanent protective group at the O4-position, respectively.

2. Nucleoside derivative according to claim 1, wherein, when R⁴ ≠ H, R¹, R² and R³ are each H.

3. Nucleoside derivative according to claim 1, wherein, when R² = OCH₃, R³ = H.

4. Nucleoside derivative according to any of claims 1-3, wherein R⁴ = CH₃.

5. Nucleoside derivative according to any of claims 1 to 4, wherein the common functional group for preparing oligonucleotides is a phosphite amide of the formula
NC-CH₂-CH₂-O-P-N(Q)₂, p-NC-C₆H₄-CH₂-CH₂-O-P-N(Q)₂, P-NO₂-C₆H₄-CH₂-CH₂-O-P-N(Q)₂ or CH₂ = CH-CH₂-O-P-N(Q)₂,
at position R⁵,
wherein the Q-groups can be identical or different and can represent linear or branched alkyl residues having 1 to 4 C-atoms.

6. Nucleoside derivatives according to claim 5, wherein the Q-group is ethyl or isopropyl.

7. Nucloside derivative according to any of claims 1-6, wherein R⁸ in ΨR⁸ at position R⁶ is O-alkyl, O-alkenyl, O-acetal, or O-silylether, when Ψ = O, or O-alkyl, when Ψ = S.

8. Nucleoside derivative according to claim 1, wherein halogen is Cl, Br or I.

9. Method for preparing nucleoside derivatives according to any of claims 1-8,
(a1) reacting a compound of the general formula (II) 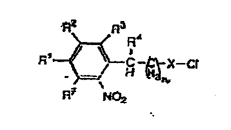 wherein R', R², R³, R⁴, R⁷, n and X have the meaning as mentioned above,
with N-methylimidazole, pyridine, N,N-dimethylaminopyridine, triazole or tetrazole, or
(a2) converting a compound of the general formula (V) 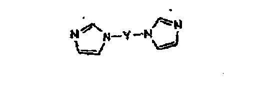 Y = C = O or C = S
with a methylating agent and reacting the obtained product with an alcohol and, subsequently
(b) reacting the acylating reagent (IV) of step (a1) or (a2) 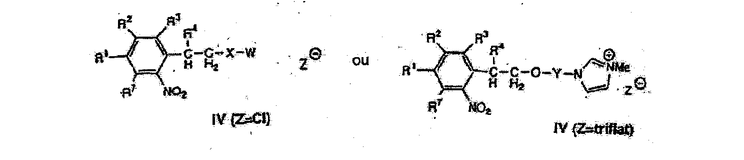 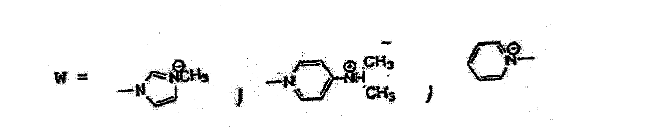 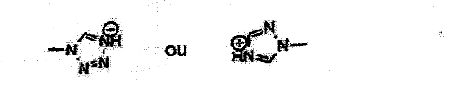 with a nucleoside of the general formula (IX) 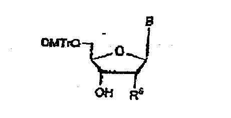 wherein R⁵, R⁶ and B have the meaning as mentioned above.

10. Method according to claim 9, wherein a phosphite amide group of the formula NC-CH₂-CH₂-O-P-N(Q)₂, p-NC-C₆H₄-CH₂-CH₂-O-P-N(Q)₂, p-NO₂-C₆H₄-CH₂-CH₂-O-P-N(Q)₂ or CH₂ = CH-CH₂-O-P-N{Q)₂,
is introduced at the 5' position of the generated nucleoside derivatives, wherein the Q-groups can be identical or different and can represent linear or branched alkyl groups having 1 to 4 C-atoms.

11. Method according to claim 9 or 10, wherein step (a1) or (a2) are carried out in a polar organic solvent at temperatures between -10 and +10°C.

12. Method according to claim 11, wherein the polar solvent is dichloromethane.

13. Method according to claim 11 or 12, wherein the temperature is about 0°C.

14. Method according to any of claims 9 to 13, wherein step (b) is carried out in dichloromethane or in a solvent mixture containing dichloromethane at temperatures of about 0°C.

15. Method according to any of claims 9 to 14, providing in step (b) the acylating reagent on step (a) in dichloromethane and adding the nucleoside drop-wise.

16. Use of a nucleoside derivative having photo-labile protective groups of the general formula (I) 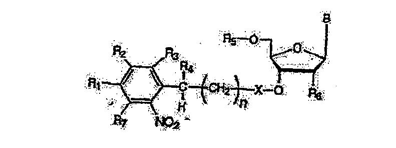 having
R¹ = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R² = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R³ = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R⁴ = H, NO₂, CN, OCH₃, halogen, alkyl, alkoxy, or alkoxyalkyl residue having 1 to 4 C-atoms, or an aryl, optionally substituted, or an aliphatic acyl having 2 to 5 atoms;
R⁵ = H, dimethoxytrityl (DMTr), or a protective group common in nucleotide chemistry, or a protective group common for preparing oligonucleotides;
R⁶ = H, OH, halogen or ΨR⁸, wherein Ψ = O or S and R⁸ = alkyl or Alkoxyalkyl residue having 1 to 4 C-atoms, or an optionally substituted aryl, or an aliphatic acyl having 2 to 5 atoms, and a protective group common in nucleotide chemistry R⁷ = H, NO₂, CN, OCH₃, halogen, alkyl, alkoxy, or alkoxyalkyl residue
having 1 to 4 C-atoms, or an aryl, optionally substituted, or an aliphatic acyl having 2 to 5 atoms;
n = 0 or 1
X = SO₂, OC(O) or OC(S);
B = H, adenine, cytosine, guanine, thymine, uracil, 2,6-diaminopurine-9-yl, hypoxanthine-9-yl, 5-methylcytosine-1-yl, 5-amino-4-imidazole carboxylic acid-1-yl, or 5-amino-4-imidazole carboxylic acid amide-3-yl, wherein, when B = adenine, cytosine or guanine, the primary amino function has optionally a temporary or permanent protective group, or thymine or uracil have a permanent protective group at the O4-position, respectively.
for preparing oligonucleotides or nucleic acid chips.

17. Nucleic acid chips, prepared by using a nucleoside derivative as defined in claim 16, wherein the oligomers, which are prepared by a light-controlled synthesis, are bound to the solid phase via the 5'-end.

18. Use of a nucleic acid chip according to claim 17 for enzyme reactions, which start from the free 3'-hydroxyl group.

19. Use of a nucleic acid chip according to claim 17 for solid phase-supported polymerase chain reactions or sequencing.

20. Use of a nucleic acid chip according to claim 17 for ligase reactions.

21. Use of a nucleic acid chip according to claim 17 for the synthesis of cDNA.

22. Use of a nucleic acid chip according to claim 17 for the reverse transcription.

23. Use of a nucleic acid chip according to claim 17 for the PCR or multiplex PCR.

24. Use of a nucleic acid chip according to claim 17 for DNA computing.

## Revendications

1. Dérivés de nucléoside avec des groupes de protection photolabiles de la formule
générale (I) 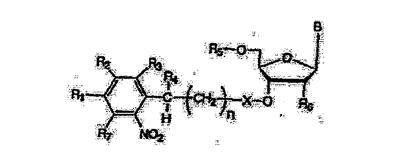 avec
R¹ = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R² = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R³= H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R⁴ = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R⁵ = H, diméthoxytrityle (DMTr) ou un groupe de protection habituel dans la chimie des nucléotides ou un groupe de protection habituel pour la production d'oligonucléotides,
R⁶ = H, OH, halogène ou ΨR⁸, Ψ = O ou S et R⁸ = alcoyle ou alcoxyalcoyle avec de 1 à 4 atomes C ou un reste d'aryle éventuellement substitué ou un reste d'acyle aliphatique avec de 2 à 5 atomes ainsi qu'un groupe de protection habituel dans la chimie des nucléotides R⁷ = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
X = SO₂, OC(O) ou OC(S)
n = 0 ou 1 pour X = SO₂
n = 1 pour X = OC(O) ou OC(S)
B = H, adénine, cytosine, guanine, thymine, uracile, 2,6-diaminopurin-9-yl, hypoxanthin-9-yl, 5-méthylcytosin-1-yl, acide 5-amino- 4-imidazolcarbon-1-yl ou amide d'acide 5-amino-4-imidazolcarbon- 3-yl, la fonction amino primaire présentant, dans le cas de B = adénine, cytosine ou guanine, éventuellement un groupe de protection temporaire ou permanent ou thymine ou uracile présentant, à la position 04, éventuellement un groupe de protection permanent.

2. Dérivé de nucléoside selon la revendication 1, dans lequel R¹, R² et R³ sont, dans le cas de R⁴ ≠ H, chacun H.

3. Dérivé de nucléoside selon la revendication 1, dans lequel R³ = H dans le cas de R² = OCH₃.

4. Dérivé de nucléoside selon l'une des revendications 1 à 3, dans lequel R⁴ = CH₃.

5. Dérivé de nucléoside selon l'une des revendications 1 à 4, dans lequel le groupe fonctionnel habituel pour la production d'oligonucléotides à la position R⁵ est un groupe phosphitamide de la formule
NC-CH₂-CH₂-O-P-N(Q)₂, p-NC-C₆H₄-CH₂-CH₂-O-P-N(Q)₂, P-NO₂-C₆H₄-CH₂-CH₂-O-P-N(Q)₂ ou CH₂ = CH-CH₂-O-P-N(Q)₂, les groupes Q pouvant être identiques ou différents et signifiant des restes d'alcoyle linéaires ou ramifiés avec de 1 à 4 atomes C.

6. Dérivé de nucléoside selon la revendication 5, dans lequel le groupe Q est éthyle ou isopropyle.

7. Dérivé de nucléoside selon l'une des revendications 1 à 6, dans lequel le reste R⁸ dans le groupe ΨR⁸ à la position R⁶ est, dans le cas de Ψ = O, un groupe O-alcoyle, O-alkényle, O-acétal ou O-silyléther ou, dans le cas de Ψ = S, un groupe O-alcoyle.

8. Dérivé de nucléoside selon la revendication 1, dans lequel halogène signifie Cl, Br, I.

9. Procédé de production de dérivés de nucléoside selon l'une des revendications 1 à 8, dans lequel
(a1) une liaison de la formule générale (II) 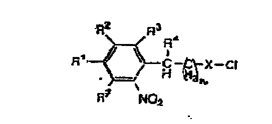 dans R¹, R², R³, R⁴, R⁷, n et X ont la signification citée ci-dessus,
se transforme avec N-méthylimidazol, pyridine, N,N-diméthylaminopyridine, triazol ou tétrazol, ou
(a2) une liaison de la formule générale (V) 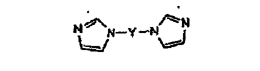 Y=C=OouC=S
se transforme avec un agent de méthylation ainsi que fait réagir le produit contenu avec un alcool et ensuite
(b) fait réagir le réactif d'acylation (IV) à l'étape (a1) ou (a2) 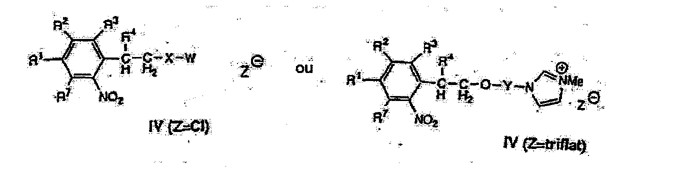 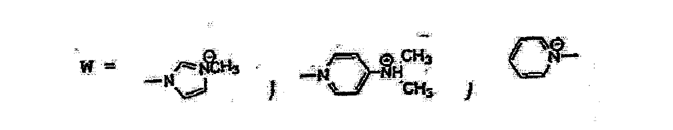 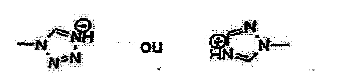 Y = C=O ou C=S avec un nucléoside de la formule générale (IX) 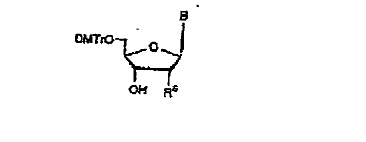 ayant, dans R⁵, R⁶ et R⁸, la signification indiquée plus haut.

10. Procédé selon la revendication 9, dans lequel on introduit, dans la position 5' des dérivés de nucléoside produits, un groupe phosphitamide de la formule
NC-CH₂-CH₂-O-P-N(Q)₂, p-NC-C₆H₄-CH₂-CH₂-O-P-N(Q)₂, p-NO₂-C₆H₄-CH₂-CH₂-O-P-N(Q)₂ ou CH₂ = CH-CH₂-O-P-N (Q)₂,
les groupes Q pouvant être identiques ou différents et signifiant des restes d'alcoyle linéaires ou ramifiés avec de 1 à 4 atomes C.

11. Procédé selon la revendication 9 ou 10, dans lequel on réalise l'étape (a1) ou (a2) dans un solvant organique polaire à des températures comprises entre -10 et +10 °C.

12. Procédé selon la revendication 11, dans lequel la solution polaire est dichlorométhane.

13. Procédé selon la revendication 11 ou 12, dans lequel la température est d'environ 0°C.

14. Procédé selon l'une des revendications 9 à 13, dans lequel on réalise l'étape (b) dans du dichlorométhane ou un mélange de solvant contenant du dichlorométhane à des températures d'environ 0°C.

15. Procédé selon l'une des revendications 9 à 14, dans lequel on présente, à l'étape (b), l'agent d'acylation formé à l'étape (a) dans du dichlorométhane et on ajoute goutte à goutte le nucléoside.

16. Utilisation d'un dérivé de nucléoside mit avec des groupes de protection photolabiles de la formule générale (1) 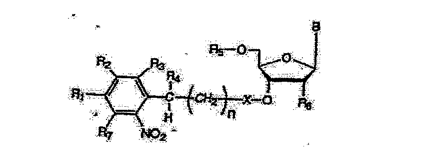 avec
R¹ = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R² = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R³ = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R⁴= = H, NO₂, CN, OCH₃, halogène, reste d'alcoyle, d'alcoxy ou d'alcoxyalcoyle avec de 1 à 4 atomes de C ou un reste d'aryle éventuellement substitué ou un reste d'acyle aliphatique avec de 2 à 5 atomes
R⁵ = H, diméthoxytrityle ou un groupe de protection habituel dans la chimie des nucléotides ou un groupe de protection habituel pour la production d'oligonucléotides,
R⁶ = H, OH, halogène ou ΨR⁸, Ψ = O ou S et R⁸ = alcoyle ou alcoxyalcoyle avec de 1 à 4 atomes C ou un reste d'aryle éventuellement substitué ou un reste d'acyle aliphatique avec de 2 à 5 atomes ainsi qu'un groupe de protection habituel dans la chimie des nucléotides
R⁷ = H, NO₂, CN, OCH₃, halogène, reste d'alcoyle, d'alcoxy ou
d'alcoxyalcoyle avec de 1 à 4 atomes de C ou un reste d'aryle éventuellement substitué ou un reste d'acyle aliphatique avec de 2 à 5 atomes
n = 0 ou 1
X= SO₂, OC(O) ou OC(S)
B = H, adénine, cytosine, guanine, thymine, uracile, 2,6-diaminopurin-9-yl,
hypoxanthin-9-yl, 5-méthylcytosin-1-yl, acide 5-amino- 4-imidazolcarbon-1-yl ou amide d'acide 5-amino-4-imidazolcarbon- 3-yl, la fonction amino primaire présentant, dans le cas de B = adénine, cytosine ou guanine, éventuellement un groupe de protection temporaire ou permanent ou thymine ou uracile présentant, à la position 04, éventuellement un groupe de protection permanent,
pour la construction d'oligonucléotides ou de puces d'acide nucléique.

17. Puce d'acide nucléique, réalisée à l'aide d'un dérivé de nucléoside selon la revendication 16, dans laquelle les oligomères construits par une synthèse contrôlée par de la lumière sont couplés, par l'extrémité 5', à la phase solide.

18. Utilisation d'une puce d'acide nucléique selon la revendication 17 pour des réactions enzymatiques partant d'un groupe 3'-hydroxyle libre.

19. Utilisation d'une puce d'acide nucléique selon la revendication 17 pour des réactions de polymérase assistées par des phases solides ou séquençage.

20. Utilisation d'une puce d'acide nucléique selon la revendication 17 pour des réactions de ligase.

21. Utilisation d'une puce d'acide nucléique selon la revendication 17 pour la synthèse de cADN.

22. Utilisation d'une puce d'acide nucléique selon la revendication 17 pour la transcription inverse.

23. Utilisation d'une puce d'acide nucléique selon la revendication 17 pour PCR ou PCR multiplexe.

24. Utilisation d'une puce d'acide nucléique selon la revendication 17 pour le calcul d'ADN.
